# EUROPÄISCHE PATENTSCHRIFT

(11) **EP 3 060 189 B1**
(45) Veröffentlichungstag und Bekanntmachung des Hinweises auf die Patenterteilung: **09.05.2018**
(21) Anmeldenummer: 14795974.6
(22) Anmeldetag: 24.10.2014
(51) Int. Cl.: A61H 35/00

(54) **EINTAUCHVORRICHTUNG**
IMMERSION DEVICE
DISPOSITIF D'IMMERSION

(30) Priorität: 24.10.2013 DE 102013017524
(43) Veröffentlichungstag der Anmeldung: 31.08.2016
(73) Patentinhaber: BSN medical GmbH, 20253 Hamburg (DE)
(72) Erfinder: SUSCHEK, Christoph, V, 40764 Langenfeld (DE)
(74) Vertreter: Jostarndt Patentanwalts-AG
(86) Internationale Anmeldenummer: PCT/EP2014/072844
(87) Internationale Veröffentlichungsnummer: WO 2015/059273

(56) Entgegenhaltungen:
- WO-A1-02/074223
- WO-A2-03/003989
- US-A1- 2013 096 490
- OPLANDER C ET AL: "A new method for sustained generation of ultra-pure nitric oxide-containing gas mixtures via controlled UVA-photolysis of nitrite solutions", NITRIC OXIDE: BIOLOGY AND CHEMISTRY, ACADEMIC PRESS, AMSTERDAM, NL, Bd. 23, Nr. 4, 15. Dezember 2010 (2010-12-15), Seiten 275-283, XP027430288, ISSN: 1089-8603, DOI: 10.1016/J.NIOX.2010.08.001 [gefunden am 2010-08-14]

## Beschreibung

Die vorliegende Erfindung betrifft eine Eintauchvorrichtung, die eine Stickstoffmonoxid-generierende Einheit und eine zum Eintauchen von Objekten vorgesehene Volumeneinheit umfasst, und die Verwendung dieser Vorrichtung zur Behandlung von Erkrankungen, insbesondere von chronischen Wunden und Diabetes- sowie Gefäßerkrankungs-assoziierten Durchblutungsstörungen.

Die Behandlung von Durchblutungsstörungen und den daraus resultierenden chronischen Wunden ist im klinischen Alltag nach wie vor unzulänglich. Diese Beschwerden sind nicht nur ein ernstes medizinisches, sondern auch ökonomisches Problem. So wird geschätzt, dass alleine in der BRD etwa 2,4 Millionen Diabetiker unter einer Durchblutungsstörung und/oder einer gestörten Wundheilung leiden. Die Lebensqualität der Kranken ist eingeschränkt, und sie leiden unter vermeidbaren Schmerzen. Die Behandlungskosten werden auf 3 Milliarden Euro jährlich geschätzt. Und mit der Alterung der Bevölkerung werden künftig noch weit mehr Menschen an solchen schwer heilenden Wunden leiden. So gehen Schätzungen von einer Verdopplung der Zahlen bis 2025 aus.

Die aktuelle Therapie stützt sich vor allem auf die mäßig effiziente pharmakologische Unterstützung der Gewebedurchblutung, sowie auf eine für die Heilung chronischer Wunden unzureichende Unterstützung durch Wundauflagensysteme.

Ein wichtiges physiologisches Prinzip der menschlichen Haut ist die enzymatische Produktion von Stickstoffmonoxid mit Hilfe von Enzymen aus der Familie der NO-Synthasen, die von allen Zelltypen synthetisiert werden können [1]. Das Substrat der NO-Synthasen ist die Aminosäure L-Arginin. Man unterscheidet heute zwischen zwei konstitutiv exprimierten und einer induzierbaren Isoform der NO-Synthasen. Zu den konstitutiv exprimierten NO-Synthasen zählen die überwiegend neuronal lokalisierte NO-Synthase (nNOS) und die vorwiegend endothelial lokalisierte NO-Synthase (eNOS), die jedoch auch in dermalen Fibroblasten und im Hautmuskelschlauch exprimiert wird, wohingegen die induzierbare Isoform, die iNOS, erst durch die Wirkung von proinflammatorischen Stimuli induziert wird und im Gegensatz zu den konstitutiven Isoformen über einen längeren Zeitraum (Tage) lokal hohe Konzentrationen von NO produzieren kann.

Im Folgenden werden die Begriffe Stickstoffmonoxid, Stickstoffoxid, Stickstoffmonoxidradikal, NO und NO• als gleichbedeutende Begriffe für das gleiche Molekül benutzt.

Daneben kann das NO auch nicht-enzymatisch aus Nitrit oder Nitrosothiolen freigesetzt werden. Die nicht-enzymatische NO-Generierung findet unter sauren und reduzierenden Bedingungen statt. Dabei wird z. B. aus Nitrit NO freigesetzt. Physiologisch bedeutsam ist diese Reaktion im sauren Milieu des Magens sowie der Haut. Es ist zudem bekannt, dass UV_{A}-Licht aus Nitrit eine Substanz mit den physiologischen Eigenschaften von NO freisetzen kann [2]. Tatsächlich konnte demonstriert werden, dass NO über den Weg des lichtenergieinduzierten Zerfalls aus Nitrit entstehen kann [3; 4].

NO steuert im Rahmen entzündlicher Prozesse der Haut, im Zusammenspiel der unterschiedlichen Zellsysteme u. a. die Proliferation, die Differenzierung von Hautzellen und somit z. B. auch die Wundheilung [5]. In Wundheilungsprozessen der Haut sind eine Reihe von Genen dominant als NO-reguliert identifiziert worden [6; 7] und entsprechend stellte sich die Wundheilung in iNOS-defizienten Mäusen als ein signifikant verzögerter Prozess dar [8]. Weitere Gene, die unter der Transkriptionskontrolle durch NO stehen, sind protektiv wirkende Stress-Schutz-Gene wie Heat-Shock-Proteine, Chaperone oder auch die Hämoxygenase-1. Ein weiterer Teil von NO-regulierten Genen dient entweder der Gegenregulation der Entzündungsreaktion oder der Reparatur von lokalen Schäden (hierzu zählen insbesondere viele Mitglieder der Familie der Matrix-Metalloproteinasen (MMP)). NO kann die Genexpression der MMPs, aber auch ihrer physiologischer Inhibitoren, der tissue inhibitors of matrix proteinases (TIMP), beeinflussen und überdies durch Nitrosierung deren Aktivität modulieren und somit einem vermehrten Kollagenabbau durch die MMPs entgegenwirken [9]. Daneben beeinflusst NO auch die Expression und Aktivität von Wachstumsfaktoren, wie z. B. dem VEGF [37; 38]. So konnte z. B. die Angiogenese, neben der Kollagensynthese eine Schlüsselkomponente der Wundheilung, durch NO-Donoren angeregt werden [39], wobei NO in Keratinozyten und Makrophagen die Synthese des Angiogenesefaktors VEGF zu induzieren vermag [5; 40].

Zudem wurde in Versuchen mit exogenen NO-Donoren gezeigt, dass NO zu einer signifikanten Erhöhung der Kollagensynthese in Fibroblasten führt [10; 11]. Ein wichtiger physiologischer Induktor der Kollagenneusynthese ist der transforming growth factor-β (TGF- β), wohingegen Interleukin-1 (IL-1), IL-6, TNF-α sowie reaktive Sauerstoffspezies (ROS) die Kollagenneusynthese signifikant mindern oder sogar hemmen können [12; 13]. NO kann auch aufgrund seiner Fähigkeit, mit anderen Radikalen zu reagieren und diese so zu eliminieren, protektiv wirken [14]. So ist NO in der Lage, vor hydroxylradikalinduzierten DNA-Schäden und vor H₂O₂-induziertem Zelltod zu schützen und es besitzt eine größere Kapazität zur Terminierung der radikalinduzierten Lipidperoxidation als Vitamin E [15; 16].

Daneben werden auch zahlreiche andere protektive Eigenschaften von NO beschrieben. So soll NO vor hypoxieinduzierten Schäden schützen, es entfaltet hepatozyten- und neuroprotektive Wirkungen und über eine Inaktivierung von Effektorcaspasen kann es vor Apoptose schützen [17]. Zudem kann NO bereits in geringen Konzentrationen wichtige Komponenten des antioxidativen Schutzes wie z. B. den Glutathionstoffwechsel (GSH) modulieren, indem es eine Erhöhung der Expression der beiden Schlüsselenzyme der GSH-Synthese, der γ-Glutamyl-Cystein-Synthetase (γ-GCS) und der γ-Glutamyl-Transpeptidase induziert [18].

Einmal gebildet, diffundiert NO leicht sowohl in die Gefäßwand als auch in das Gefäßlumen und ist z. B. beteiligt an der Regulation der Thrombozytenadhäsion und Thrombozyten-aggregation, des vaskulären Rollings sowie der Transmigration von neutrophilen Granulozyten und Monozyten, als auch der endothelialen Permeabilität [20]. NO relaxiert zudem in der Gefäßwand die glatten Muskelzellen über eine Aktivierung der löslichen Guanylatzyklase, dem Schlüsselenzym in der Regulation des Blutdrucks. Das endothelial gebildete NO hat somit eine essentielle Bedeutung für die Aufrechterhaltung sowohl der Gefäßfunktion als auch der Gefäßstruktur und beeinflusst somit wesentlich hämodynamische Parameter, insbesondere den Blutdruck, aber auch gewebsischämische Zustände [21; 22].

Bei einer verminderten NO-Syntheserate wird im Tiermodell eine verzögerte Gefäßneubildung und Wundheilung sowie eine stark gestörte Reepithelisierung von Hautwunden aufgrund einer verminderten Proliferationsrate der Keratinozyten beobachtet. Als Transmitter der Gefäßrelaxation kann NO die Blutflussrate im Wundareal erhöhen und somit zu einer vermehrten Sauerstoff- und Nährstoffversorgung sowie einer gesteigerten zellulären Infiltration des Gewebes führen [5].

Die topische Behandlung von Wunden mit NO-Donoren, während der frühen Phase der kutanen Wundheilung führt zu einer signifikant beschleunigten Wundschließung und Reepithelisierung in der Ratte [23], wie auch zu einer verbesserten Wundheilung bei Mäusen mit diabetischen Hintergrund [24]. Auch die tägliche topische Exposition von Wunden mit NO-haltigem Luft-Plasma verbesserte signifikant die Wundheilung septischer sowie aseptischer Wunden im Rattenmodell [25]. Trotz der zahlreichen Hinweise auf den positiven Einfluss von NO auf die Wundheilung wurde am Menschen bis dato nur eine Pilotstudie mit einem 55-Jährigen Patienten dokumentiert, bei dem eine NO-Gas Therapie zur vollständigen Heilung eines mehrere Jahre alten, therapieresistenten Ulcus cruris venosum am Fuß führte [26]. Exogen verabreichtes NO kann durch den Abbau von reaktiven Sauerstoffspezies Ischämie/Reperfusion-bedingte Schäden mildern und die Mikrozirkulation des Hautgewebes wesentlich verbessern. Diese Eigenschaften spielen eine besondere Rolle in der Revitalisierung von Randgebieten von freien Lappenplastiken im Rahmen von Weichteilgewebsdeckungen [26]

Aktuelle, den NO-Haushalt-betreffende Therapieansätze versuchen überwiegend die durch NO induzierte cGMP-abhängige Signalkaskade zu adressieren. Therapieansätze zu direkten Beeinflussung der NO-Verfügbarkeit im Organismus begrenzen sich auf die Verwendung organischer Nitrite und Nitrate [27]. NO-Gas wird in der Klinik bis dato nur inhalativ in der Therapie verschiedener akuter Lungendysfunktionen verwendet, wobei in experimentellen Studien auch eine systemische Wirkung inhalierten NOs nachgewiesen werden konnte [28]. Der Diffusionskoeffizient von NO bei 37 °C ist ca. 1,4-fach höher als der von Sauerstoff oder Kohlenmonoxid, wonach eine für das NO-Molekül erreichbare Diffusionsstrecke in Geweben von 500 µm errechnet wurde [29].

Ghaffari et al. konnte signifikante antibakterielle Wirkungen und somit die Relevanz in der Behandlung von mit Bakterien infizierten Wunden und Brandverletzungen sowie nicht heilenden Wunden durch exogenes NO-Gas nachweisen [31; 32], wobei die verwendeten NO-Konzentrationen in vitro keine toxische Effekte auf humane Fibroblasten, Keratinozyten oder Endothelzellen zeigten [33].

Zusammenfassend hat sich hiermit das Stickstoffmonoxid (NO) als physiologisch wichtiges bioaktives Molekül herausgestellt. Durch seine erweiternde Wirkung auf die Blutgefäße, die zudem sehr schnell auftritt, besitzt das NO eine große Bedeutung für die Blutversorgung von Organen. Darüber hinaus spielt das NO auch in anderen physiologischen Prozellen eine Rolle als wichtiger Botenstoff. So schützt das NO als Radikalfänger vor Hypoxie-induzierten Schäden und moduliert wichtige Komponenten des antioxidativen Schutzes. Beachtlicherweise steuert das NO bei entzündlichen Prozessen der Haut im Zusammenspiel der unterschiedlichen Zellsysteme beispielsweise die Proliferation und Differenzierung von Hautzellen und fördert so die Wundheilung.

In entsprechender Weise zeigt sich im Tiermodell, dass eine verminderte NO-Syntheserate mit einer verzögerten Gefäßneubildung und Wundheilung einhergeht.

Basierend auf den Erkenntnissen zu NO gibt es bereits Ansätze gasförmiges NO für die Therapie von Durchblutungsstörungen oder chronischen Wunden einzusetzen. Bisher wird ein für therapeutische Zwecke genutztes NO-haltiges Gas aus Gasflaschen (Industriegas) bereitgestellt, deren Lagerung und Handhabung in einer Klinik oder einer anderen Therapieeinrichtung aufgrund der erforderlichen Sicherheitsmaßnahmen aufwändig ist. Dies gilt insbesondere für eine mobile Vorrichtung. Zudem muss die Qualität des gelagerten Gases für medizinische Anwendungen hohen Anforderungen genügen, die den Aufwand für Herstellung und Lagerung weiter erhöhen. Schon eine geringe Verunreinigung des Gases führt zur Bildung unerwünschter und eventuell giftiger Nebenprodukte. Die europäischen Arzneimittel- und Gesundheitsbehörden haben demgemäß hohe Anforderungen an die Reinheit des zu verwendenden Stickstoffmonoxids gestellt. Neben der Verwendung von "technischen" NO-Gasen für die medizinische Anwendung, gibt es Verfahren zur plasmachemischen Erzeugung von Stickstoffmonoxid. Diese Verfahren erfordern nachgeschaltete, teilweise sehr aufwändige, Reinigungsverfahren und es ist schwierig eine optimale Konzentration von NO für den jeweiligen therapeutischen Zweck einzustellen.

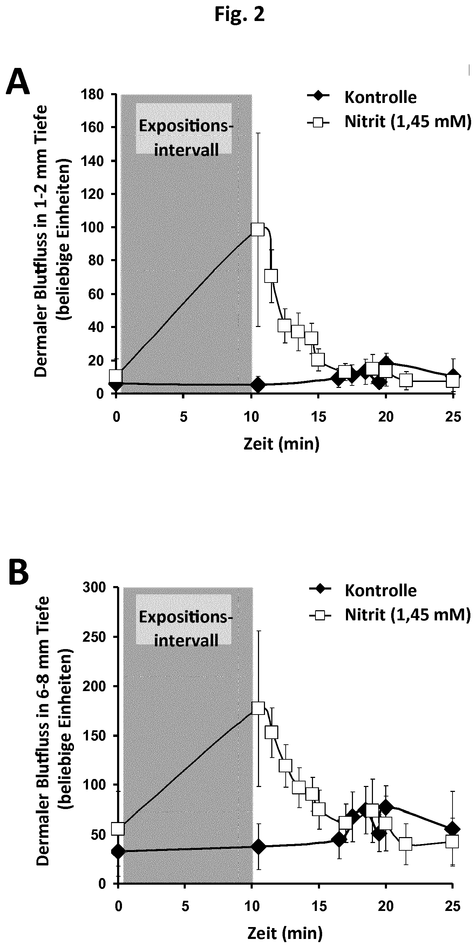

Opländer et al. 2010 (Nitric Oxide Biology & Chemistry, 23: 275-283) beschreiben eine Methode zur kontinuierlichen Bildung hochreiner NO-Gasgemische über die kontrollierte UVA-Photolyse von Nitritlösungen. Sie lehrt hierbei in Figur 2 zwei Apparaturen die durch die UVA-Bestrahlung einer Nitritlösung ein NO-haltiges Gasgemisch erzeugen, das am Ausgang der Apparaturen (Kennzeichnung mit "->CLD") anfällt. Diese Vorrichtungen sind nicht als Eintauchvorrichtung ausgestaltet.

Die WO 2002/074223 betrifft einen Fußmassage-Apparat und offenbart hierbei einen abnehmbaren Wasserkessel 130 mit einer oberhalb angebrachten UV-Lampe 225, die zur Sterilisierung des Wassers dienen soll. Die WO'223 sieht allerdings keine Verbindung zwischen Wasserkessel und Fußbadeeinheit vor. Ein weiterer Nachteil der WO'223 besteht darin, dass sie keine Umwälz- oder Pumpvorrichtung vorsieht, mit Hilfe derer die wässrige Lösung von dem Wasserkessel in das Fußbad und wieder zurück geleitet werden kann und damit einen geschlossenen Kreislauf zwischen den beiden Kompartimenten ermöglichen kann.

Die WO 2003/003989 offenbart ein Gerät zur Wundheilung und Infektionskontrolle und lehrt ein Gehäuse 12, in das ein Fuß 100 eingeführt werden kann, und wobei durch eine UV-Lampe 14 das in dem Gehäuse eingebrachte photoaktivierbare Material u.a. zur Bildung von NO stimuliert wird. Das NO-haltige Gasgemisch kann dann durch einen Ventilator ("Fan 50") in dem Gehäuse zirkulieren. Gemäß der WO'989 fehlt es an einer Trennung zwischen der NO-generierenden Einheit und der Eintauchvorrichtung und diese ist auch nicht zur Aufnahme einer wässrigen Lösung als Trägermedium geeignet. Weiterhin kann bei der WO'989 der Körperteil nicht kontrolliert temperiert behandelt werden.

Die Druckschrift US 2013/096490 offenbart System zur Wundbehandlung und lehrt gemäß Paragraph [0031] ein Fußbad mit einem Behandlungskessel 800, der eine Kammer 810 zur Aufnahme des zu behandelten Körperteils aufweist. Die Kammer ist mit einem Deckel verschließbar, der auf der Innenseite LEDs aufweist (s. [0011]). Diese als Beleuchtung gedachten LEDs würden bei einer Photolyse von NODs das NO direkt im Behandlungskessel erzeugen und damit dem Benutzer der Gefahr einer Exposition gengenüber gasförmigem NO aussetzen.

Es besteht daher noch Bedarf an neuen Verfahren zur Behandlung von Durchblutungsstörungen und chronischen Wunden.

Aufgabe der Erfindung ist es daher, einen neuen therapeutischen Ansatz zur Behandlung von Durchblutungsstörungen und chronischen Wunden bereitzustellen, der bezüglich mindestens einer der oben genannten Nachteile verbessert ist.

### Zusammenfassung der Erfindung

Diese Aufgabe wird gemäß der Erfindung dadurch gelöst, dass eine Eintauchvorrichtung bereitgestellt wird, die folgendes umfasst:
a. eine Stickstoffmonoxid (NO) - generierende Einheit (SGE) (1) mit einer UV-Strahlungsquelle (6) und einer Volumenkammer (3) zur Aufnahme einer NODenthaltenden wässrigen Lösung, wobei die SGE so ausgestaltet ist, dass mittels UV-Strahlung die wässrige Lösung mit NO angereichert werden kann, und
b. eine Volumeneinheit VE (2) zum Eintauchen von Objekten, wobei die VE die mit NO angereicherte, von der SGE (1) transferierte wässrige Lösung aufnehmen kann,
wobei die Volumeneinheit (2) ein nach oben offener Behälter ist, und die SGE (1) dicht verschließbar ist, dadurch gekennzeichnet, dass
der Transfer der wässrigen Lösung durch zwei, die SGE und VE verbindende Öffnungen (5) in einer gemeinsamen Wand oder durch zwei Leitungen erfolgt und die wässrige Lösung mit Hilfe einer Umwälz- bzw. Pumpvorrichtung durch die Volumenkammer (3) der SGE (1) geleitet werden kann und wieder in die VE (2) ausgeleitet werden kann, so dass die Volumenkammer (3) der SGE (1) und die Volumeneinheit (2) einen geschlossenen Kreislauf für die wässrige Lösung bilden, und
wobei die Eintauchvorrichtung mit einer Temperiervorrichtung versehen ist, die durch Heizen oder Abkühlen eine Einstellung einer ausgewählten Temperatur erlaubt, und
wobei die UV-Strahlungsquelle (6) ausgewählt ist aus der Gruppe enthaltend eine mit entsprechenden Fluorochromen beschichtete Glüh- oder Gasentladungslampe (niedrigdruck- oder hochdruckentladend), eine Licht-emittierende Diode (LED), eine organische Licht-emittierende Diode (OLED), und ein Laser.

Die erfindungsgemäße Eintauchvorrichtung vereinigt mehrere entscheidende Vorteile gegenüber den aus dem Stand der Technik bekannten therapeutischen Ansätzen.

Die Abtrennung der NO-generierenden Einheit von der Volumeneinheit, in die die Objekte eingetaucht werden können, geht mit einer erhöhten Sicherheit einher. So können die eventuell gesundheitsschädlichen oder beeinträchtigenden Stickstoffmonoxidvorstufen selektiv in der SGE vorgehalten werden, ohne dass sie mit den einzutauchenden Objekten in Berührung kommen und lediglich das NO wird über die Verbindung zwischen SGE und VE übertragen.

Bei dem erfindungsgemäß vorteilhaften Verfahren der photochemischen NO-Generierung, die bevorzugt mittels UV-Strahlung arbeitet, ist die UV-Quelle somit einfach abzuschirmen und der Benutzer vor der Exposition gegenüber schädlichen UV-Strahlen geschützt.

Weiterhin erlaubt diese Abtrennung auch, dass der Gehalt und die Reinheit des in die VE übertretenden NO-haltigen Trägermediums kontrolliert und auch reguliert werden kann und im Extremfall (z.B. durch ein Sicherheitsventil) die Verbindung dieser beiden Einheiten komplett unterbrochen werden kann.

Üblicherweise erschwert die kurze Halbwertszeit des NO den therapeutischen Einsatz. Mit der erfindungsgemäßen Vorrichtung kann trotz der kurzen Halbwertszeit durch eine kontinuierliche NO-Nachsynthese ein gleichbleibender NO-Spiegel aufrechterhalten werden.

Dieser Fähigkeit zur Regulierung und Steuerung ist gerade im therapeutischen Bereich von entscheidendem Vorteil, da sie eine auf den jeweiligen Patienten abgestimmte Behandlung ermöglicht.

Der modulare Aufbau ermöglicht auch den Einsatz von NOD-Nachfüllbehältern (z.B. in Form von Kartuschen), die eine reproduzierbare und sichere Herstellung von NO gewährleisten können.

Durch einfache Anpassung der Volumeneinheit bezüglich Größe, Form und Material können unterschiedlichste NO-abhängige Eintauchanwendungen realisiert werden, von der Einwirkung auf Produkte wie Gerätschaften und Instrumente bis hin zur therapeutischen Behandlung von menschlichen und tierischen Organismen unter Eintauchen von Körperteilen oder des ganzen Körpers.

Da die NO-generierende Einheit einen Bestandteil der erfindungsgemäßen Eintauchvorrichtung darstellt, kann auf eine externe Zuführung von NO, wie es meist durch Gasflaschen geschieht, verzichtet werden.

Dies erlaubt den Einsatz als mobiles System, das gerade im therapeutischen Bereich eine Anwendung außerhalb von Praxen oder Kliniken ermöglicht und damit insbesondere bei chronischen Krankheiten mit einer kostengünstigeren Behandlung und einer höheren Patienten-Compliance einhergeht.

Bei der erfindungsgemäßen Eintauchvorrichtung handelt es sich um eine einfach aufgebautes Gerät mit handelsüblichen Komponenten so dass es nicht nur kostengünstig in der Herstellung ist, sondern auch bei geringer Fehleranfälligkeit einfach in der Anwendung ist.

Zusammenfassend stellt das erfindungsgemäße Eintauchvorrichtung eine NO-basierte Therapieform dar, bei der preiswert, zuverlässig, sicher und für den Patienten individualisierbar NO-haltige Gemische erzeugt werden können.

### Die Erfindung im Einzelnen

Erfindungsgemäß stellt die Erfindung eine Eintauchvorrichtung bereit, die folgendes umfasst:
Eine Stickstoffmonoxid (NO) - generierende Einheit (SGE) mit einer UV-Strahlungsquelle und einer Volumenkammer zum Eintauchen von Objekten und insbesondere von Rumpfabschnitten, Extremitätenabschnitten oder ganzen Körpern, wobei die Volumenkammer als Trägermedium ein Lösungsmedium aufnehmen kann, in der mittels UV-Strahlung aus dieser UV-Strahlungsquelle aus Stickstoffmonoxidvorstufen (NOD) durch Spaltung NO erzeugt wird und das Lösungsmedium als Eintauchmedium mit dem NO angereichert wird.

Erfindungsgemäß stellt die Erfindung eine Eintauchvorrichtung bereit, die die folgenden Einheiten enthält und insbesondere aus den folgenden Einheiten besteht:
a. eine Stickstoffmonoxid (NO) - generierende Einheit (SGE) mit einer UV-Strahlungsquelle und einer Volumenkammer zur Aufnahme eines NODenthaltenden Trägermediums, wobei die SGE so ausgestaltet ist, dass mittels UV-Strahlung das Trägermedium mit NO angereichert werden kann,
b. und eine Volumeneinheit VE zum Eintauchen von Objekten, wobei die VE das mit NO angereicherte, von der SGE transferierte Trägermedium aufnehmen kann.

Erfindungsgemäß ist die Volumeneinheit der erfindungsgemäßen Eintauchvorrichtung ein nach oben offener Behälter. Hierdurch kann in einfacher Weise die Volumeneinheit mit dem Eintauchmedium als Trägermedium befüllt werden und das einzutauchende Objekt von oben in die Volumeneinheit und damit das Eintauchmedium getaucht werden.

Erfindungsgemäß ist die SGE der Eintauchvorrichtung dicht verschließbar. Auf diese Weise kann in dieser, für die NO-Generierung zuständigen Einheit kein NO in die Umwelt gelangen, sondern es wird selektiv in das Trägermedium überführt und steht damit vollständig der (therapeutischen) Eintauchanwendung zur Verfügung. Diese Ausgestaltung ist gerade im Rahmen der therapeutischen Anwendung von großem Vorteil, da der Anwender keiner unnötigen NO-Exposition ausgesetzt wird.

Erfindungsgemäß ist das Lösungsmedium der SGE mit dem Eintauchmedium der VE identisch. In dieser Form kann das mit NO angereicherte Lösungsmedium von der SGE in die VE geleitet werden und in bevorzugter Weise von dort wieder in die SGE zurückgeleitet werden, so dass für dieses Medium ein geschlossener Kreislauf existiert. Hierdurch kann das "verbrauchte" Eintauchmedium durch Zirkulation und erneuter NO-Anreicherung in der SGE wieder "regeneriert" werden.

Erfindungsgemäß enthält bei der Eintauchvorrichtung die SGE eine Volumenkammer, die mit der Volumeneinheit (VE) kommuniziert, wobei die Volumenkammer ein Trägermedium enthält, das mit Hilfe einer Umwälz- bzw. Pumpvorrichtung durch die Volumenkammer der SGE geleitet werden kann und wieder in die VE ausgeleitet werden kann.

Erfindungsgemäß sind die Volumenkammer der SGE und die Volumeneinheit durch eine oder mehrere Öffnungen in einer gemeinsamen Wand oder durch eine oder mehrere Leitungen assoziiert, d.h. das Trägermedium kann durch die Öffnung(en) oder die Leitung(en) von der SGE zur VE und auch wieder zurück transportiert werden.

Eine Leitung im Sinne der Erfindung kann jede Anlage zum Zweck des Transports des erfindungsgemäßen flüssigen, zähen oder gelförmigen Trägermediums sein. Beispiele für entsprechende Leitungen sind Rohre, Schläuche oder Kanäle, die zweckmäßigerweise mit der Pumpvorrichtung integriert sind.

Bevorzugterweise sind die die Volumenkammer der SGE und die Volumeneinheit durch zwei Öffnungen in einer gemeinsamen Wand oder durch zwei Leitungen assoziiert.

Erfindungsgemäß bilden die Volumenkammer der SGE und die Volumeneinheit einen geschlossenen Kreislauf für das Trägermedium, so dass das "verbrauchte" Trägermedium der VE nach Rücktransport in der SGE erneut mit NO angereichert werden kann und durch Rücktransport in die VE den gewünschte Konzentration an NO wieder herstellt. Dieser geschlossene Kreislauf ist am einfachsten durch die oben ausgeführten zwei Öffnungen, bzw. zwei Leitungen zu gewährleisten.

Erfindungsgemäß sind sowohl das Lösungsmedium als auch das Eintauchmedium als Trägermedium aufzufassen.

In einer Ausführungsform ist das Trägermedium ein organisches oder anorganisches, flüssiges, zähes bis gelartiges Trägermedium.

In einer bevorzugten Ausführungsform ist dieses Trägermedium eine wässrige Lösung und insbesondere bevorzugt eine wässrige Pufferlösung.

Das Lösungsmedium der SGE ist dadurch gekennzeichnet, dass das gasförmige, flüssige oder gelartige Trägermedium eine oder mehrere NO-Vorstufen (NOD) enthält.

In bevorzugter Weise enthält das Lösungsmedium darüber hinaus einen oder mehrere der folgenden Stoffe: Katalysatoren, Detergentien, Puffersubstanzen, Chromophore, Substanzen, die die NOD stabilisieren wie bspw. Dimethylsulfoxid oder Ethanol, Substanzen, die die Halbwertszeit von NO erhöhen, wie bspw. in der US 2003/0039697 offenbart, NOD-Stabilisatoren, Antioxidantien, Farbstoffe, pH-Indikatoren, Pflegestoffe, Duftstoffe, pharmakologisch aktive Substanzen.

In einer besonders bevorzugten Ausführungsform enthält das Trägermedium, also das Lösungsmedium und/oder Eintauchmedium, ein System, welches mehrfach oxidierte Stickoxide, Sauerstoffradikalanionen, Hydroxylradikale oder "aquatisierte Elektronen" abbaut oder neutralisiert.

In einer speziellen Ausführungsform ist hierbei das System, welches mehrfach oxidierte Stickoxide, Sauerstoffradikalanionen, Hydroxylradikale oder "aquatisierte Elektronen" abbaut oder neutralisiert, ausgewählt aus der Gruppe bestehend aus Ascorbinsäure, Ascorbat, Vitamin E, Derivate von Vitamin E, Thiole, Radikalfänger, Sauerstoffspezies oder Stickstoffspezies abbauende Enzyme.

Bei einer gepufferten Lösung als Lösungsmedium liegt der pH-Wert zweckmäßigerweise zwischen 3,0 und 10, bevorzugt zwischen 5,5 und 7,4 und besonders bevorzugt zwischen 6,0 und 7,0.

Bei dem Lösungsmedium handelt es sich bevorzugt um eine isotonische Salzlösung und besonders bevorzugt um eine isotonische gepufferte Salzlösung.

In besonders bevorzugter Weise weist das wässrige Lösungsmedium die folgende Zusammensetzung auf:
- Puffersalze zur Einstellung eines Lösung-pH von zwischen 6,0 und 7,4
- 50 - 250 mM NOD
- 50 - 250 mM Antioxidans

In spezieller Weise weist das Lösungsmedium die folgende Zusammensetzung auf:
- Phosphat-gepufferte Salzlösung (PBS) mit pH = 6,0 - 7,4
- 100 mM NOD
- 150 mM Antioxidans

In bevorzugter Weise handelt es sich bei der PBS um eine Lösung mit folgender Zusammensetzung:
- 8 g/L NaCI
- 0,2 g/L KCl
- 1,424 g/L Na₂HPO₄
- 0,2 g/L KH₂PO₄

In einer alternativen Ausführungsform wird eine Acetat-gepufferte Salzlösung mit folgender Zusammensetzung verwendet:
- 8 g/L NaCl
- 0,2 g/L KCl
- Gemisch aus Essigsäure und Natrium-Acetat mit 50 - 250 mM Endkonzentration

Die obengenannten Ausführungsformen finden für den erfindungsgemäßen Fall Anwendung, dass das Lösungsmedium identisch mit dem Eintauchmedium ist.

In bevorzugter Weise enthält das Eintauchmedium einen oder mehrere der folgenden Stoffe: Katalysatoren, Detergentien, Puffersubstanzen, Chromophore, Substanzen, die die NOD stabilisieren wie bspw. Dimethylsulfoxid oder Ethanol, Substanzen, die die Halbwertszeit von NO erhöhen, wie bspw. in der US 2003/0039697 offenbart, NOD-Stabilisatoren, Antioxidantien, Farbstoffe, pH-Indikatoren, Pflegestoffe, Duftstoffe, pharmakologisch aktive Substanzen.

Substanzen, die die Halbwertszeit von NO erhöhen, werden beispielsweise in der US-Anmeldung US 2003/0039697 offenbart, deren Offenbarung durch Verweis vollumfänglich mit in diese Anmeldung mit aufgenommen wird.

Der Fachmann wird in Hinblick auf den jeweiligen Verwendungszweck und basierend auf seinem allgemeinen Fachwissen geeignete Stoffe oder Stoffgemische auswählen. Hierbei wird er vor allem berücksichtigen, dass bei der Verwendung als Badevorrichtung physiologische verträgliche und/oder dermatologisch verträgliche Stoffe und Stoffgemische zur Anwendung kommen.

In bevorzugter Weise liegt das Eintauchmedium als gepufferte wässrige Lösung vor.

Bei einer gepufferten Lösung als Eintauchmedium liegt der pH-Wert zweckmäßigerweise zwischen 3,0 und 10, bevorzugt zwischen 5,5 und 7,4 und besonders bevorzugt zwischen 6,0 und 7,0.

Bei dem Eintauchmedium handelt es sich bevorzugt um eine isotonische Salzlösung und besonders bevorzugt um eine isotonische gepufferte Salzlösung.

In besonders bevorzugter Weise weist das wässrige Eintauchmedium die folgende Zusammensetzung auf:
- Puffersalze zur Einstellung eines Lösung-pH von zwischen 5,0 und 8,0
- 100 - 250 mM NOD
- 100 - 250 mM Antioxidans

In besonders bevorzugter Weise weist das wässrige Eintauchmedium die folgende Zusammensetzung auf:
- Puffersalze zur Einstellung eines Lösung-pH von zwischen 6,0 und 7,4
- 50-250 mM NOD
- 50 - 250 mM Antioxidans

In spezieller Weise weist das Eintauchmedium die folgende Zusammensetzung auf:
- Phosphat-gepufferte Salzlösung (PBS) mit pH = 6,0 - 7,4
- 100 mM NOD
- 150 mM Antioxidans

In bevorzugter Weise handelt es sich bei der PBS um eine Lösung mit folgender Zusammensetzung:
- 8 g/L NaCl
- 0,2 g/L KCl
- 1,424 g/L Na₂HPO₄
- 0,2 g/L KH₂PO₄

In einer alternativen Ausführungsform wird eine Acetat-gepufferte Salzlösung mit folgender Zusammensetzung verwendet:
- 8 g/L NaCl
- 0,2 g/L KCl
- Gemisch aus Essigsäure und Natrium-Acetat mit 50 - 250 mM Endkonzentration

In einer Ausführungsform der Erfindung enthält das Eintauchmedium eine oder mehrere pharmakologisch aktive Substanzen. Diese können die pharmakologische Wirkung des NOs unterstützen oder unabhängig von dem NO in einer für die entsprechende Erkrankung therapeutisch relevanten Weise wirken.

In einer Ausführungsform der Erfindung enthält das Eintauchmedium eine oder mehrere der folgenden pharmakologisch aktiven Substanzen: Entzündungshemmer wie bspw. nichtsteroidale Antirheumatika (NSAIDs) oder Corticoide, Immunsuppressiva, Antibiotika, Antikoagulantien, Antithrombotika, antivirale Agenzien, Antimykotika, Lokalanästhetika und Analgetika.

Diese weiteren pharmakologisch aktiven Substanzen können aber im Sinne einer Kombinationsbehandlung nicht nur Bestandteil des Eintauchmediums sein, sondern entweder vor oder nach dem Badevorgang zur Anwendung kommen.

In einer Ausführungsform der Erfindung wird durch die Eintauchvorrichtung ein sprudelndes Bad bereitgestellt. Dies kann durch Einblasen eines Gases erzeugt werden oder durch eine chemische Reaktion, bei der ein Gasbildner wie beispielsweise ein Carbonatsalz durch Ansäuerung der Lösung zur Freisetzung von CO₂-Gas induziert wird.

In einer weiteren Ausführungsform der Erfindung ist die Eintauchvorrichtung mit einer Vorrichtung versehen, die die Freisetzung von NO in die Umwelt verringert oder gänzlich verhindert. Dies kann eine mechanische Abtrennung sein, die beispielsweise in Form einer Haube oder eine Abdeckfolie die Volumeneinheit abdeckt, wobei sie eine Aussparung für den einzutauchenden Körperteil vorsieht. Alternativ kann es sich um eine Absaugvorrichtung handeln, die das aus dem Eintauchmedium freigesetzte NO absaugt und entweder dem Eintauch- oder dem Lösungsmedium zuführt oder das NO abbaut bzw. abfiltriert.

In einer bevorzugten Ausführungsform ist die SGE ein im Wesentlichen geschlossenes, d.h. hermetisch von der Umwelt abgeschottetes System, das lediglich mit der Volumeneinheit in Verbindung steht. Dadurch wird gewährleistet, dass das in der SGE erzeugte NO (bevorzugt selektiv) an die VE abgegeben wird und nicht in die Umwelt gelangt.

In einer weiteren Ausführungsform ist die SGE mit einem NO-Sensor gekoppelt, so dass als Rückkopplung auf den gemessenen NO-Wert das Ausmaß der NO-Generierung flexibel angepasst werden kann.

Dieser NO-Sensor als Messvorrichtung zur Quantifizierung des NO kann in der SGE, in der VE oder auf der Außenseite der Eintauchvorrichtung angebracht sein. In einer besonderen Ausführungsform sorgt die NO-Sensor-assoziierte Steuerung dafür, dass bei Überschreiten eines kritischen NO-Wertes die SGE die NO-Generierung gänzlich einstellt.

In einer Ausführungsform der Erfindung wird die SGE so angesteuert, dass in dem Eintauchmedium der Gehalt an NO über den Zeitraum der Behandlung konstant gehalten wird.

In einer alternativen Ausführungsform der Erfindung wird die SGE so angesteuert, dass der Gehalt an NO über den Zeitraum der Behandlung ansteigt oder abfällt.

In einer weiteren Ausführungsform der Erfindung wird die Eintauchvorrichtung zur Behandlung von Gegenständen, Vorrichtungen oder Instrumenten verwendet. Durch die Einwirkung von NO auf diese Gegenstände können diese gereinigt oder desinfiziert werden, die mikrobielle Belastung reduziert werden oder ein Biofilm verringert oder entfernt werden.

In einer bevorzugten Ausführungsform wird die Eintauchvorrichtung hierbei zur Reinigung oder Desinfektion von medizinischen oder chirurgischen Instrumenten verwendet.

Erfindungsgemäß wird das NO durch Photolyse generiert. Danach werden z. B. die in einer nitrithaltigen Lösung (z. B. Natriumnitrit) enthaltenen Nitrit-Ionen (NO₂⁻) mittels elektromagnetischer Strahlung (z. B. UV_{A}-Strahlung mit Wellenlängen zwischen 320 und 440 nm) gespalten (Photolyse), wodurch NO generiert wird. Unter reduktiven Bedingungen bzw. unter Schutzgas (z. B. Stickstoff) verläuft der durch elektromagnetische Strahlung induzierte Zerfall von Nitrit über unterschiedliche, zum Teil auch parallele, thermodynamisch jedoch verschieden gewichtete Kanäle. Es kann angenommen werden, dass im Kanal 1 (Reaktionen 1 bis 5) UV_{A}-Strahlung (mit einem Optimum bei 354 bis 366 nm) Nitrit zum Stickstoffmonoxidradikal (NO•) und zum Sauerstoffradikalanion (O^{•-}) spaltet (Gleichung 1). Das zuletzt genannte Produkt initiiert im Folgenden in wässrigen Lösungen die Bildung des reaktiven Hydroxylradikals (OH^{•}) (Gleichung 2). Das Hydroxylradikal reagiert mit Nitrit, was zur Bildung des Nitrogendioxidradikals (NO₂^{•}) führt (Gleichung 3). Dieses kann mit Stickstoffmonoxid zu Dinitrogentrioxid (N₂O₃) weiterreagieren (Gleichung 4).

NO₂- + hv → NO^{•}+ O^{•-} (1)

O^{•-} + H₂O → OH^{•} + OH⁻ (2)

NO₂⁻ + OH^{•} → NO₂^{•} + OH- (3)

NO₂^{•} + NO^{•} → N₂O₃ (4)

N₂O₃ + H₂O → 2 NO₂⁻ + 2 H⁺ (5)

In Kanal 2 (Gleichungen 6-10) scheinen Hydroxylradikale unter den genannten Bedingungen keine Rolle zu spielen, jedoch werden ein "aquatisiertes" Elektron (e⁻_{aq}) sowie ein Stickstoffdioxidradikal gebildet (Gleichung 6). Das Elektron wird bei einem Überschuss an Nitrit auf dieses übertragen und das daraus resultierende Nitritanion (Gleichung 7) wird in Wasser zum NO-Radikal reduziert (Gleichung 8). Die folgenden Reaktionen in Gleichungen (9) und (10) entsprechen denen in Gleichungen (4) und (5). Die Gewichtung von Kanal 1 zum Kanal 2 bildet dabei ein Verhältnis von ca. 40:60.

NO₂⁻ + hv → NO₂^{•} + e⁻_{aq} (6)

e⁻_{aq} + NO₂⁻ → NO₂⁻ (7)

NO₂⁻ + H₂O → NO^{•} + 2 OH- (8)

NO^{•} + NO₂^{•} → N₂O₃ (9)

N₂O₃ + H₂O → 2 NO₂⁻ + 2 H⁺ (10)

Wie aus den Reaktionen 1 bis 10 deutlich wird, wird der photolytische Zerfall von Nitrit von einer parallelen Produktion reaktiver und zytotoxischer, chemischer Spezies begleitet. Aus den Reaktionen in Gleichungen (4) und (9) wird zudem ersichtlich, dass NO₂-Radikale (NO₂^{•}) mit dem in Gleichung (1) gebildeten NO rückreagieren können.

Es wurde erkannt (EP1903003A1), dass durch den Einsatz von mindestens einem System, welches NO₂-Radikale oder Sauerstoffspezies abbaut oder neutralisiert, während der Generierung von Stickstoffmonoxid die Bildung der genannten reaktiven Zwischenprodukte des lichtinduzierten Nitritzerfalls (NO₂^{•}, O^{•-}, OH^{•}, e⁻_{aq}) unterbunden wird oder deren Eliminierung erfolgt, während gleichzeitig die Generierung von Stickstoffmonoxid nicht gemindert wird. Somit wird die Ausbeute frei verfügbarem NO und die Reinheit des Gases erhöht.

Der Anstieg der NO-Freisetzung sowie der hohe Reinheitsgrad beruht auf einer reaktionsbedingten Eliminierung der reaktiven Zwischenprodukte, z. B. gemäß den folgenden Reaktionen (11) bis (17).

N₂O₃ + RS⁻ → NO₂⁻ + RSNO (11)

RSNO + hv → NO^{•} + RS^{•} (12)

NO₂^{•} + RS⁻ → NO₂⁻ + RS^{•} (13)

NO^{•} + RS → RSNO (14)

BA + OH^{•} → BA-OH (15)

VitC + NO₂^{•} → NO₂⁻ + VitC^{•-} (16)

Trol + NO₂^{•} → NO₂⁻ + Trol^{•-} (17)

(Abkürzungen: RS⁻, Thiol; RSNO, S-Nitroso-Thiol; RS^{•}, Thioylradikal; BA, Benzoesäure; VitC, Vitamin C, Askorbat, Askorbinsäure; VitC^{•}, das Radikal von VitC; Trol, Trolox; Trol^{•}, das Radikal von Trolox).

Dieses Verfahren (EP1903003A1) ermöglicht durch die Präsenz dieser oder anderer funktionsgleicher Systeme während der Bildung von Stickstoffmonoxid eine hohe Ausbeute an Stickstoffmonoxid, während gleichzeitig die Bildung von unerwünschten (mehrfach) oxidierten Stickoxiden, insbesondere des NO₂^{•}, sowie von Hydroxylradikalen und reaktiven aquatisierten Elektronen effizient verhindert wird, oder diese Substanzen nach ihrer Entstehung eliminiert werden bzw. nur noch in so geringem Maße erzeugt werden können, dass sie in Lösung verbleiben und nicht in die Gasphase übertreten können. Somit können diese Substanzen z. B. keinen pathologisch relevanten Schaden in Folge der Inhalation der Inhalationsgase verursachen.

Als Systeme, welche reaktive Stickoxidspezies (z. B. Stickstoffdioxid-Radikale) oder reaktive Sauerstoffspezies abbauen oder neutralisieren, werden vorzugsweise reaktive Sauerstoffspezies oder Stickoxidspezies (ROS oder RNS)-abbauende bzw. - neutralisierende Substanzen (Antioxidantien) verwendet. Weiter bevorzugt handelt es sich um Askorbinsäure, Askorbat, Vitamin E und seine Derivate, Thiole, andere Antioxidantien, Radikalfänger oder ROS- und RNS-abbauend Enzyme.

Dem Fachmann sind zahlreiche Antioxidantien bekannt, die er entsprechend des jeweiligen Trägermediums und des Mechanismus zur NO-Generierung auswählen wird.

Für ein lipophiles Trägermedium, wie es durch ein organisches Lösungsmittel bereitgestellt werden kann, eignen sich beispielsweise Antioxidantien wie Tocopherole, Tocotrienole, Tocomonoenole, Butylhydroxyanisol (BHA) und Butylhydroxytoluol (BHT).

Für ein hydrophiles Trägermedium, bevorzugt hierbei wässrige Lösungen, eignen sich insbesondere organische schwefelhaltige Verbindungen wie Glutathion, Cystein, oder Thiomilchsäure oder auch organische Säuren wie Ascorbinsäure, alpha-Liponsäure, Hydroxyzimtsäuren wie p-Cumarsäure, Ferulasäure, Sinapinsäure oder Kaffeesäure, oder Hydroxybenzoesäuren wie Gallussäure, Procatechusäure, Syringasäure oder Vanillinsäure.

Andere bevorzugte Antioxidantien umfassen polyphenolische Verbindungen wie Anthocyane, Flavonoide und Phytoöstrogene.

Es hat sich darüber hinaus herausgestellt, dass die Bindung oder Eliminierung der genannten reaktiven Zwischenprodukte des lichtinduzierten Nitritzerfalls (NO₂^{•}, O^{•-}, OH^{•}, e⁻_{aq}) auch in einem neutralen pH-Bereich erfolgen kann, wobei aus Nitrit eine maximale NO-Freisetzung in einer maximalen Reinheit erreicht werden kann.

Unter sauren Bedingungen (pH < 7,0) wird in wässrigen Lösungen der "spontane" Nitritzerfall begünstigt. Entsprechend der Gleichungen 18-20 befindet sich das Nitritanion (NO₂⁻) in wässrigen Lösungen in einem Gleichgewicht mit seiner konjugierten Säure, der salpetrigen Säure (HNO₂). HNO₂ seinerseits befindet sich im Gleichgewicht mit Distickstofftrioxid (N₂O₃), welches spontan zu NO^{•} und NO₂^{•} zerfällt.

NO₂⁻ + H⁺ ⇆ HNO₂ (18)

2 HNO₂ ⇆ N₂O₃ + H₂O (19)

N₂O₃ ⇆ NO^{•} + NO₂^{•} (20)

In einer Ausführungsform eines beschriebenen Verfahrens (EP1903003A1) erfolgt damit die UVA-induzierte Generierung von Stickstoffmonoxid vorzugsweise in einem pH-Intervall von 0 bis 12, insbesondere von 1 bis 10, besonders bevorzugt von 1,5 bis 6, speziell von 2 bis 6, ganz speziell von 2,5 bis 4.

Der Gehalt an NO in dem Lösungsmedium und/oder Eintauchmedium beträgt zwischen 10 µM und 5 mM, bevorzugt zwischen 50 µM und 2 mM und besonders bevorzugt zwischen 100 µM und 200 µM.

Je nach eingesetzter Nitrit- oder Antioxidanskonzentration sowie je nach Höhe des eingesetzten physikalischen Dekompensationsreizes, der zum Zerfall von Nitrit führt, kann durch das erwähnte Verfahren (EP1903003A1) eine hohe Konzentration an Stickstoffmonoxid erreicht werden.

In einer Lösung kann die Menge des generierten Stickstoffmonoxids durch die verwendete Konzentration der das Stickstoffmonoxid freisetzenden Agenzien und durch die physikalische und/oder chemische Induktion, welche für die Freisetzung von Stickstoffmonoxid aus den Agenzien verantwortlich ist, gesteuert werden.

Dabei werden unter dem Begriff "physikalische und/oder chemische Induktion" neben der Intensität der elektromagnetischen Strahlung sowie der Expositionsdauer, welcher die Reaktionslösung ausgesetzt wird, im Allgemeinen auch die Reaktionsparameter verstanden, welche einen Einfluss auf die Bildung von Stickstoffmonoxid an sich und auf die Konzentration an Stickstoffmonoxid haben. Hierzu zählen im Allgemeinen der pH-Wert der Reaktionslösung, der Redoxstatus der Reaktionslösung, die Temperatur der Reaktionslösung, die exponierte Bestrahlungsfläche, die Zeit der Einwirkung einer Induktionsgrößen auf die Stickstoffmonoxid freisetzenden Agenzien, die Entfernung der Quelle der elektromagnetischen Strahlung zur Reaktionslösung, das Spektrum der elektromagnetischen Strahlungsquelle, die Absorptions-, Transmissions-, Reflexionseigenschaften der Reaktionslösung, die Konzentration von biologischen oder chemischen Katalysatoren oder Vermittlersubstanzen, die auch außerhalb der "typischen" physiko-chemischen Bedingungen einer optimalen NO-Freisetzung eine solche aus NO-generierenden Substanzen durch Katalyse oder entsprechende Akzeptoreigenschaften dennoch ermöglichen. Insbesondere sind damit Chromophore und andere Substanzen gemeint, mit deren Hilfe z. B. auch elektromagnetische Strahlung außerhalb des UV_{A}-Spektrumbereiches in der Lage sein könnte aus den entsprechenden NO-bildenden Agenzien eine NO-Freisetzung zu ermöglichen.

So ist es beispielsweise bei konstant gehaltenen Induktionsgrößen durch den Einsatz variierender Konzentrationen der Stickstoffoxid-freisetzenden Substanz(en) möglich, variierende Mengen an Stickstoffmonoxid freizusetzen.

Ferner ist es bei einer konstanten Konzentration der Stickstoffoxid-freisetzenden Substanz(en) möglich, die Freisetzung von Stickstoffmonoxid durch Variation der Einstellparameter der jeweiligen Induktionsgrößen zu verändern. Bei einer konstant gehaltenen Induktionsgröße können daher durch den Einsatz hoher Konzentrationen der NO-freisetzenden Substanzen hohe NO-Mengen freigesetzt werden und umgekehrt. Bei einer konstanten Konzentration der NO-freisetzenden Substanz kann die NO-Generierung durch Variation der Einstellparameter der jeweiligen Induktionsgrößen verändert werden. Die Einstellparameter können dabei alternativ oder gleichzeitig zur Regulation der NO-Erzeugung herangezogen werden. Insbesondere über die gleichzeitige Regelung der NO-Erzeugung über mehrere Einstellparameter kann das Verfahren vorteilhafterweise hinsichtlich der NO-Erzeugung sowie der Erzeugung unerwünschter Nebenprodukte optimiert werden.

Die Substanz, welche zur Freisetzung von Stickstoffmonoxid verwendet und in dem erfindungsgemäßen Verfahren verwendet wird, unterliegt grundsätzlich keiner Beschränkung, solange sie in der Lage ist, unter dem Einfluss von elektromagnetischer Strahlung Stickstoffmonoxid freizusetzen. Sie kann beispielsweise aus der Gruppe bestehend aus
(a) reinen Stoffen oder Stoffgemischen, welche unter dem Einfluss elektromagnetischer Strahlung Stickstoffmonoxid generieren;
(b) Stoffgemische, die zusätzlich zu den unter (a) genannten Stoffen oder Stoffgemischen Hilfssubstanzen enthalten, welche ausgewählt sind aus der Gruppe, bestehend aus Photoakzeptoren, Photoamplifier, Übergangsmetalle, insbesondere Kupferionen, Enzyme oder Katalysatoren, um spontan oder unter physikalischem oder chemischem Einfluss Stickstoffmonoxid generieren; und
(c) Stoffe oder Stoffgemische, die erst als Folge einer zuvor stattgefundenen chemischen Reaktion unter Zuhilfenahme der unter (a) genannten Stoffe und gegebenenfalls der unter (b) genannten Hilfssubstanzen unter dem Einfluss von elektromagnetischer Strahlung spontan oder unter physikalischem oder chemischem Einfluss Stickstoffmonoxid generieren, ausgewählt werden.

Ferner können die unter (a) beschriebenen Substanzen zusätzlich durch Temperaturveränderungen und/oder Feuchtigkeitsveränderungen und/oder pH-Veränderungen ihrer Lösungen und/oder Veränderungen des Redoxstatus ihrer Lösungen Stickstoffmonoxid freisetzen.

In einer bevorzugten Ausführungsform der Erfindung sind die Stickstoffmonoxidvorstufen (NOD) ausgewählt aus der Gruppe enthaltend organische Nitrate, anorganische Nitrate, Nitrite, Schwefel-, Stickstoff- oder Sauerstoff-Nitrosoverbindungen, NO-MetallVerbindungen und NO-chelatierende Substanzen.

Stickstoffmonoxidvorstufen sind im Stand der Technik bekannt und dem Fachmann geläufig.

Beispiele für NOD umfassen Diazeniumdiolate (z.B. US Patente Nr. 7,105,502; 7,122,529; 6,673,338), trans[RuCI([15]aneN4)NO]+2, Nitrosyl-Liganden, 6-Nitrobenzo[a]pyrol, S-Nitroso-Glutathion, S-Nitroso-Thiol, , Nitroanilin-Derivate (siehe US 2013/0224083), 2-Methyl-2-Nitrosopropan, Imidazoyl-Derivate, Hydroxylnitrosamin, Hydroxylamin und Hydroxyharnstoff.

In einer Ausführungsform der Erfindung werden das Eintauchmedium und/oder das Lösungsmedium mittels eines Nachfüllbehälters eingesetzt. Hierbei kann das fertig formulierte Medium durch den Behälter passend in die Eintauchvorrichtung eingesetzt werden und durch die herstellungsseitig vorgegebene Formulierung wird gewährleistet, dass die therapeutisch optimale Formulierung vorliegt.

In einer weiteren Ausführungsform werden die Inhaltstoffe des Eintauchmediums und/oder des Lösungsmedium in bevorzugt vorportionierter Form (sog. Verpackungseinheit) dem Medium hinzugegeben. Da die erfindungsgemäße NO-Generierung auch mit herkömmlichem Leitungswasser möglich ist, kann so der Anwender auf dieses Leitungswasser zurückgreifen und mit den Inhaltsstoffen, die beispielsweise Puffersubstanz, Salze, NOD und Antioxidans umfassen, mischen und so zu einem gebrauchsfertigen Lösungs- bzw. Eintauchmedium gelangen.

Bei der vorportionierten Form liegen die Inhaltsstoffe bevorzugt in fester Form vor. So können sie als Puder, Pulver, Granulat, Tablette, Filmtablette, Dragee, Weichgelatinekapsel, Hartgelatinekapsel, Oblong, Caplet, Brausetablette oder Pille vorliegen, wobei die Verpackungseinheit zweckmäßigerweise die für jeweils eine Behandlung ausreichenden Menge enthält.

In einer bevorzugten Ausführungsform liegen die Inhaltsstoffe als Brausetablette vor. In dieser Form werden sie schnell gelöst und reichern das Medium zudem mit dem entsprechenden -bevorzugt inerten- Gas (bspw. CO₂) an. Diese Darreichungsform ist zudem im Bereich der Badeanwendungen den Anwendern wohlbekannt und besitzt daher auch eine hohe Compliance.

Alternativ können die Inhaltsstoffe in flüssiger oder halbfester Form vorliegen. Halbfeste Formen umfassen beispielsweise: Suspension, Emulsion, Paste, Creme, Salbe, Gel oder Lotion. Die Vorportionierung als Verpackungseinheit kann beispielsweise durch die Verpackung in Ampullen, Flaschen, Säckchen oder Tuben gewährleistet werden.

In einer weiterhin bevorzugten Ausführungsform ist die Verpackungseinheit so ausgestaltet, dass sie von der Form her eine fehlerfreie Anwendung in der Eintauchvorrichtung ermöglicht. So ist die Form in bevorzugter Weise als Kartusche ausgestaltet, die nur in einer Orientierung in die Eintauchvorrichtung befestigbar ist. Darüber hinaus kann diese Kartusche mit einem Arretierungsmechanismus ausgestaltet sein, der nur nach korrekter Arretierung in der Eintauchvorrichtung die Inhaltsstoffe in der gewünschten Weise freigibt. Zweckmäßigerweise kann die Eintauchvorrichtung hierbei mit einem Sensor ausgestattet sein, der eine inkorrekte Orientierung bzw. Arretierung der Kartusche detektiert und dem Anwender anzeigt.

In einem weiterem Aspekt stellt die Erfindung einen Kit bereit, der eine Verpackungseinheit für eine Behandlung umfasst, wobei diese Verpackungseinheit eine pulverförmige, gelförmige oder flüssige Zusammensetzung enthaltend NOD, Puffersubstanz, Antioxidans und optional ein Lösungsmittel aufweist.

Die Freisetzung kann ausgehend von wässrigen Nitrit- oder S-Nitrosothiol-Lösungen erfolgen. Dabei ist aus praktischen Gründen die Verwendung einer wässrigen Lösung von Natriumnitrit oder S-Nitrosothiolen als NO-Quelle bevorzugt. Die wässrige Lösung kann eine Konzentration der NO-Vorstufen von vorzugsweise 0,001 bis 10000 mM, insbesondere 0,2 bis 6000 mM, besonders bevorzugt 0,3 bis 5000 mM, speziell 0,4 bis 2000 mM, ganz speziell 0,5 bis 1500 mM, aufweisen.

In einer weiteren Ausführungsform weist die wässrige Lösung als Eintauch- und/oder Lösungsmedium eine Konzentration der NO-Vorstufen von zwischen 1 µM bis 5000 mM, bevorzugt von zwischen 100 µM bis 2000 mM, besonders bevorzugt von zwischen 500 µM bis 500 mM und ganz speziell zwischen 1 mM und 150 mM auf.

Die Art der Bestrahlung von NO-generierenden Ausgangssubstraten ist dem auf dem vorliegenden Gebiet tätigen Fachmann an sich bekannt. Es kann jedwede elektromagnetische Strahlung verwendet werden, welche in der Lage ist, photolabile NO-Derivate unter Bildung von Stickstoffmonoxid zu zersetzten. Beispielsweise kann im Rahmen der vorliegenden Erfindung die Herstellung von Stickstoffmonoxid mittels photolytischer Spaltung unter Verwendung einer UV_{A}-Strahlung mit Wellenlängen von beispielsweise 320 bis 440 nm erfolgen. Es kann jedoch auch elektromagnetische Strahlung jeder anderen Wellenlänge verwendet werden, die allein oder unter Zuhilfenahme chemischer, physikalischer oder biologischer Verfahren eine direkte, durch andere Hilfsstoffe induzierte bzw. erleichterte oder katalysierte photolytische Spaltung von NO-generierender NO-Vorstufen (NO-Derivate) induziert.

Die Herstellung von Stickstoffmonoxid kann auch in Lösungen erfolgen, die mit inerten Gasen gesättigt sind. In solchen mit inerten Gasen (Stickstoff (N₂), Helium (H₂), Argon, usw.) gesättigten Lösungen hat das darin gelöste NO eine wesentlich längere Lebensdauer und kann auch in höheren Konzentrationen in Lösung verbleiben. Allgemein wird angenommen, dass die maximale Löslichkeit von NO in wässrigen Lösungen ca. 2 mM beträgt. In diesem Zusammenhang können als wässrige Lösungen auch Kulturmedien oder Infusionsmedien bzw. -Puffer, Serum, Blut, Gele und alle anderen Stoffe verstanden werden, die in der Lage sind, Gase aufzunehmen.

Das durch Photolyse von photolabilen NO-Vorstufen hergestellte Stickstoffmonoxid kann beispielsweise zu Inhalationszwecken eingesetzt werden. Weitere spezifische Anwendungsgebiete sind die Anregung des Stoffwechsels von Geweben durch äußerliche Anwendung, die strukturelle Modifikation organischer sowie anorganischer Flächen, die Sterilisation oder die Erzeugung von Zytotoxizität. Das durch Photolyse erzeugte Stickstoffmonoxid kann auch zur Begasung von Wunden, insbesondere zur Wundheilung chronischer, nicht-heilender, eventuell Bakterien-befallener Wunden eingesetzt werden. Das Stickstoffmonoxid kann, wenn es in gesättigten Flüssigkeiten erzeugt wird, auch systemisch zur Behandlung von Bluthochdruck eingesetzt werden. Schließlich kann das Stickstoffmonoxid auch in mit Stickstoffmonoxid nitrosierte Träger erzeugt werden, welche spontan wieder NO freisetzen. Das Stickstoffmonoxid kann auch zur Herstellung unterschiedlicher NO-bindender Substanzen (z. B. NO-Donoren) genutzt werden.

Die Qualität eines auch in Lösungen gelagerten bzw. eingebrachten Gases für medizinische Anwendungen muss hohen Anforderungen genügen. Schon eine geringe Verunreinigung des Gases führt zur Bildung unerwünschter und eventuell giftiger Nebenprodukte. Die Entstehung dieser Nebenprodukte bei längerer Lagerung von Stickstoffmonoxid-enthaltenden Gasflaschen sowie bei der plasmatechnischen Herstellung von Stickstoffmonoxid und die Entfernung dieser Radikale stellen einen großen technischen sowie finanziellen Nachteil dar. Die Vorteile des photolytischen Verfahrens zur Herstellung von Stickstoffmonoxid-haltigen Lösungen sind die Einfachheit der Herstellungsmethode des NO-haltigen Gases, der besonders hohe Reinheitsgrad des hergestellten NO-Gasgemisches, geringe Folgekosten und keine Lagerungskosten, die besonders einfache Handhabung der NO-Erzeugung sowie der Reinheitskontrolle, das unvergleichlich günstige Verhältnis der Herstellungskosten zu der Menge des hergestellten NO-Gases.

### Erfindungsmäßige Vorrichtung

Für eine äußerliche Anwendung kann NO in Form eines NO-haltigen Gases oder Plasmas angewendet werden, sowie in Form eines spontan oder induziert zerfallenden NO-Donors. Die hier vorliegende Erfindung betrifft eine medizinische Badevorrichtung, die in der Lage ist, in einem Lösungsmedium, z. B. in einer Badelösung, gelöste Stickstoffmonoxid-Vorstufen mittels physikalisch-chemischer Reize zu spalten und mit dem so erzeugtem Stickstoffmonoxid das Lösungsmedium, z. B. die Badelösung, anzureichern, sodass die Vorrichtung und die Badelösung zur Unterstützung medizinischer Therapien bei Tier und Mensch sowie zu Anreicherung unterschiedlicher Medien mit NO verwendet werden kann.

In einer Ausführungsform der Erfindung wird die Eintauchvorrichtung zur Behandlung von Erkrankungen verwendet. Hierbei werden in zweckmäßiger Weise Rumpfabschnitte, Körperabschnitte oder ganzen Körper in das NO-haltige Eintauchmedium eingetaucht.

Die erfindungsgemäße Eintauchvorrichtung kann somit nicht nur zur Behandlung von chronischen oder akuten Erkrankungen eingesetzt werden, sondern auch zur möglichen Prävention solcher Erkrankungen. Sofern nicht anders ausgeführt, umfasst der Begriff "Therapie" oder "Behandlung" alle Maßnahmen zur Linderung, Heilung oder Prävention der hier relevanten Erkrankungen.

Der erfindungsgemäßephysikalische Reiz für die Spaltung von NO-Vorstufen ist dabei die elektromagnetische Strahlung, also ein Reiz, der in der Lage sind, entsprechende photolabile NO-Vorstufen photolytisch unter Freisetzung von NO zu spalten.

Aufgrund des limitierten Lösungsverhaltens von NO können in entsprechenden Lösungen physiologisch-relevante NO-Konzentrationen generiert werden, die jedoch weit unter denen liegen, die für den Menschen gesundheitliche Schäden verursachen könnten. Ferner kann durch einen direkten Kontakt der menschlichen Körperoberfläche mit den NO-haltigen Lösungen eine wesentlich genauere NO-Behandlung erreicht werden als z. B. mit NO-haltigen Gasgemischen bzw. spontan zerfallenden NO-Donoren. Weiterhin stellt die Möglichkeit, dass die Vorrichtung je nach Beladung mit den entsprechenden NO-Vorstufe von unterschiedlichen Endverbrauchern benutzt werden kann, von Laien bis hin zum Fachmann, den wesentlichen Vorteil der erfindungsmäßigen Vorrichtung gegenüber anderen NO-basierten Therapien dar.

Die erfindungsmäßige Vorrichtung besteht aus einem volumenaufnehmenden Behälter (im Folgenden **VE** = *volumenaufnehmende Einheit* genannt), der das Lösungsmedium oder Medienlösungen, z. B. Badelösungen, aufnehmen kann sowie einer weiteren technischen Einheit (im Folgenden als **SGE** = *Stickstoffmonoxid-generierende Einheit* genannt), die in dem Lösungsmedium Stickstoffmonoxid generieren und somit das Lösungsmedium der VE mit NO anreichern kann.

Es besteht jedoch auch die Möglichkeit, dass die SGE nicht direkt in dem Lösungsmedium der VE das NO generiert, sondern in einer anderen Lösung, die dann mit dem Lösungsmedium der VE in gewünschter Verdünnung vermischt wird. Als Alternative kann auch ein in der SGE generiertes NO-haltiges Gasgemisch oder eine aus einer Gasflasche bereitgestelltes NO-haltiges Gasgemisch in das Lösungsmedium der VE eingeleitet werden und somit das Lösungsmedium der VE, so z. B. eine Badelösung, mit NO angereichert werden.

In eine solche mit NO angereicherte Badelösung kann dann zu therapeutischen Zwecken eine Stelle am Körper eingetaucht und gebadet werden. Dabei kann es sich um Stellen am Rumpf oder Extremitäten von Menschen oder Tieren handeln. Da die VE je nach Anwendungswunsch ein Volumen von 0,001 Liter bis 1000 Liter und mehr haben kann, lassen sich kleine Rumpf- und Extremitätenabschnitte behandeln sowie ganze Körper eintauchen, wobei eine solche Behandlung zwischen wenigen Sekunden und vielen Stunden dauern kann.

In bevorzugter Weise wird der Körper, bzw. der zu behandelnde Körperteil für 5 bis 30 Minuten, bevorzugt 7.5 bis 20 Minuten und besonders bevorzugt für 10 bis 15 Minuten in die das Eintauchmedium eingetaucht.

Ein derartiges Bad wird in bevorzugter Weise mehrfach täglich angewendet, wobei eine 2 bis 3-malige tägliche Anwendung bevorzugt ist.

Zur Steuerung der Behandlungsdauer kann die Eintauchvorrichtung in bevorzugter Weise eine Zeitsteuerungseinheit beinhalten, die nach einer fest vorgegebenen oder bevorzugt flexibel programmierbaren Zeit die NO-Generierung abschaltet.

Darüber hinaus kann das Eintauchmedium einen Farbstoff enthalten, der nach einer bestimmten Zeit eine Farbveränderung erfährt, so dass der Benutzer über das Ende des Behandlungszeitraums informiert ist.

Weiterhin kann die Eintauchvorrichtung auch eine Vorrichtung zur Messung der Durchblutung umfassen, der anhand des Therapieerfolges eine besonders gute Steuerung der Behandlungsdauer und/oder Behandlungsintensität erlaubt. Dem Fachmann sind zahlreiche Vorrichtungen zur Messung der Durchblutung bekannt. Beispiele hierfür sind Gefäßtachometer, oder der in der WO 97/46853 offenbarte Mikrosensor. Dieser Sensor umfasst einen Indikator-durchlässigen Einsatz, der in einer Öffnung eines Indikator-Behälters, der durch ein Behältnis gebildet ist, angeordnet ist, wodurch der Einsatz einen durchlässigen Wand-Abschnitt des Behälters bildet.

Als Surrogatparameter für die Hautdurchblutung können weitere vaskulär bedingte Messparameter wie die Rötung der Haut oder die Hauttemperatur dienen, für die entsprechende Messmethoden und -geräte aus dem Stand der Technik bekannt sind.

Die SGE generiert das Stickstoffmonoxid für die Anreicherung von z. B. Badelösungen durch photolytische Spaltung von Stickstoffmonoxid-vorstufen. Dabei kann die SGE ein integraler, fest mit der VE verbundener Bestandteil der Gesamtvorrichtung sein. Alternativ kann die SGE auch als eine selbstständige, externe, nicht an der VE angebrachte Einheit sein.

Ein wesentliches Charakteristikum der SGE ist jedoch, dass sie selbst ebenfalls ein eigenes Volumen hat, welches mit dem Volumen der VE verbunden ist, und dass das Volumen der SGE mit all seinem möglichen Inhalt dem entsprechenden physikalischen Reiz ausgeliefert werden kann, um NO aus chemischen NO-Vorstufen zu generieren. Die SGE kann alternativ jedoch auch dazu genutzt werden, von außen eingeleitete, NO-haltige Gasgemische zur Herstellung von mit NO-angereicherten Lösungen, z. B. Badelösungen zu generieren.

Im Folgenden wird als Beispiel für die erfindungsgemäße Ausführungsmöglichkeit der erfindungsgemäßen Vorrichtung elektromagnetische Strahlung als der relevante physikalische Reiz zur Spaltung chemischer NO-Vorstufen besprochen.

Elektromagnetische Strahlung wirdvon einer Lichtquelle emittiert, die innerhalb der SG-Einheit angebracht ist. Wichtig ist, dass die Lichtdurchflutung des SGE-Inhalts mitsamt den das Stickstoffmonoxid freisetzenden Reaktionssubstanzen im Sinne eines induzierten Stoffzerfalls bzw. einer Freisetzung von Stickstoffmonoxid maximal ist. Die Quelle der elektromagnetischen Strahlung ist dabei eine mit entsprechenden Fluorochromen beschichtete Glüh- oder Gasentladungslampe (niedrigdruck- oder hochdruckentladend), eine Licht-emittierende Diode (LED), eine organische Licht-emittierende Diode (OLED) oder ein LASER.

Für eine optimale Spaltung der in der Badelösung gelösten photolabilen NO-Vorstufen kann die das Volumen der SGE bestrahlende Lichtquelle elektromagnetische Strahlung mit Wellenlängen von 100 bis 2000 nm emittieren oder elektromagnetische Strahlung jeder anderen Wellenlänge emittieren, die allein oder mit Unterstützung chemischer, physikalischer oder biologischer Verfahren eine Spaltung von Stickstoffmonoxid-Vorstufen und dadurch eine Freisetzung von Stickstoffmonoxid induzieren kann.

Vorzugsweise sollte daher die SGE aus einem Material aufgebaut sein, das die Eigenschaften des für eine optimale Freisetzung von Stickstoffmonoxid notwendige Energie einer elektromagnetischen Strahlungsquelle nicht beeinflusst oder aufgrund seiner Eigenschaften, die für eine lichtinduzierte Freisetzung von Stickstoffmonoxid notwendigen Lichteigenschaften erst schafft oder optimiert oder im Falle der pH-abhängigen NO-Generierung den pH-induzierten Nitritzerfall fördert und optimiert.

Erfindungsgemäß umfasst die SGE eine Volumenkammer, die ein Trägermedium mit Stickstoffmonoxidvorstufen (NOD) enthält und zudem eine Vorrichtung zur Generierung des NO aus dem mindestens einen NOD.

Dies kann eine Vorrichtung zur Zugabe von Säure zu der Volumenkammer sein, wo dann pH-induziert NO generiert wird.

Erfindungsgemäß ist die SGE mit einer UV-Strahlungsquelle versehen, deren UV-Strahlung durch photolytischen Zerfall das NO direkt in dem Trägermedium generiert. Dies hat den Vorteil, dass das Trägermedium in einem abgeschlossenen Kompartiment vorliegen kann und zudem die NO-Generierung in kontrollierter und reproduzierbarer Weise ablaufen kann.

Bevorzugterweise wird zur NO-Generierung das Trägermedium in der SGE in einem flachen Behältnis von der Strahlungsquelle angestrahlt.

So ist für die photolytische Spaltung ein Behältnis mit einer Schichtdicke von zwischen 1 und 20 mm, bevorzugt von zwischen 2.5 und 10 mm und besonders bevorzugt von zwischen 5 und 7.8 mm geeignet. Es zeigte sich, dass eine entsprechend dimensionierten Schichtdicke durch optimale Ausnutzung der UV-Strahlung zu einer hohen Ausbeute an NO führt.

Zweckmäßigerweise ist das Material des Behältnisses für UV-Strahlung durchlässig. Aufgrund seiner Kenntnis der UV-Durchlässigkeit wird der Fachmann die geeigneten Materialien für das Trägermedium enthaltende Behältnis auswählen. Bei UV-Strahlung im UV_{A}-Bereich (315 bis 380 nm) kann noch herkömmliches Natron-Kalk-Glas verwendet werden, bei höherenergetischer Strahlung bis 290 nm kann Borosilikatglas zum Einsatz kommen, und bei UV-Strahlung unterhalb von 290 nm ist Quarzglas geeignet.

Auch UV-durchlässige Kunststoffe wie Polymethylpenten (PMP), modifiziertes Polymethylmethacrylat (PMMA), modifiziertes Polyvinylbutyral (Trosivol UV+®) können als Material für das Behältnis verwendet werden.

In einer bevorzugten Weise ist das Behältnis so ausgeformt, dass es mit seiner der Strahlungsquelle zugewandten Fläche einen definierten, gleichbleibenden Abstand aufweist. Bei einer röhrenförmigen Strahlungsquelle ist das Behältnis entsprechend als Hohlzylinder ausgeformt, in dessen Zentrum die Röhre positioniert ist. Das Trägermedium wird hierbei zweckmäßigerweise an einem Ende des Zylinders zugeführt, strömt über die Länge des Zylinders an der UV-Strahlungsquelle vorbei, wobei es sich zunehmend mit NO anreichert und wird am anderen Ende des Zylinders entnommen, um der VE zugeführt zu werden.

Alternativ kann das Behältnis auch ein Rohr sein, das als Spirale mit definiertem Innendurchmesser ausgeformt ist, wobei die röhrenförmige UV-Quelle im Zentrum der Spirale angeordnet ist. Diese Anordnung ermöglicht einen graduellen Anstieg der NO-Konzentration, wobei die NO-Ausbeute hier bei gleichbleibender Strahlungsintensität durch die Fließgeschwindigkeit in der Spirale gesteuert werden kann.

In einer alternativen Ausgestaltung ist bei einer flächenförmigen Strahlungsquelle (z.B. durch ein LED-Panel) das Behältnis als flächenförmiger Kasten ausgeformt. Dieser weist bevorzugt diametral angebrachten Zu- und Abflüsse für das Trägermedium auf und kann im Innern auch Trennwände enthalten, die den Fluss des Trägermediums in geeigneter Weise steuern können.

In einer weiteren Ausführungsform ist das Behältnis auf der von der Strahlungsquelle abgewandten Weise mit einer UV-reflektierenden Beschichtung versehen. Damit kann die Strahlungsausbeute zusätzlich erhöht werden, indem das reflektierte UV-Licht erneut das Trägermedium durchqueren kann und hierbei NO photolytisch generieren kann. Dem Fachmann sind entsprechende UV-reflektierende Schichten wie bspw. Aluminium oder dielektrische Schichten bekannt. In einer alternativen Ausführungsform ist die UV-reflektierende Beschichtung nicht auf dem Behältnis selbst, sondern getrennt dazu angebracht, z.B. auf der Innenwand der SGE.

In einer Ausführungsform wird die photolytische NO-Generierung vor der Behandlung als sogenannter Vorlauf durchgeführt, um in einem Zeitraum von maximal 30 Minuten, vorzugsweise von 10 bis 15 Minuten, besonders bevorzugt in weniger als 10 Minuten, die therapeutisch notwendige NO-Konzentration aufzubauen.

Zum Zwecke der Anreicherung der in der VE enthaltenen Badelösung mit NO kann die Badelösungen der VE durch das Volumen der SGE geleitet werden, dort der aus der Lichtquelle der SGE emittierten elektromagnetischen Strahlung ausgesetzt und wieder in die das Hauptvolumen fassende VE der Vorrichtung eingeleitet werden. Diese zwischen der VE und SGE stattfindende Volumenbewegung erfolgt durch eine Pumpeinheit, wobei die Umwälzungsvorrichtung ein Bestandteil der SGE oder VE sein kann oder gänzlich als externer Bestandteil der Vorrichtung fungieren kann.

Dem Fachmann sind Pump- bzw. Umwälzvorrichtungen aus dem Stand der Technik bekannt und er kann anhand der relevanten Parameter wie Viskosität des Trägermediums, erforderliche Pumpleistung, Volumen der VE und der SGE die passende Vorrichtung auswählen.

Als Pumpvorrichtungen kommen hier beispielsweise in Betracht: Schlauchpumpen, Membranpumpen, Kolbenpumpen, magnetgekoppelte Pumpen und Impellerpumpen.

Die Eintauchvorrichtung ist erfindungsgemäß mit einer Temperiervorrichtung versehen. Diese erlaubt durch Heizen und/oder Abkühlen eine Einstellung einer ausgewählten Temperatur. Die Temperatur ist einer der Parameter, die die NO-Ausbeute und die Löslichkeit des generierten NO bestimmen. Zudem kann so bei der Badeanwendung eine für die therapeutische Anwendung optimale Badetemperatur eingestellt werden. Dies kann eine für den Anwender angenehme Temperatur zwischen 23 und 28°C sein, oder eine Temperatur zwischen 5 und 15°C, die dadurch die Durchblutung der Haut steigert.

Dem Fachmann sind Temperiervorrichtungen aus dem Stand der Technik bekannt und er kann anhand der relevanten Parameter wie Volumen der Flüssigkeit und Aufheiz- und Abkühlgeschwindigkeiten die passende Vorrichtung auswählen.

Erfindungsgemäß ist eine Temperiervorrichtung insbesondere in Kombination mit einer (UV)-Strahlungsquelle erforderlich, da diese zu einem Aufheizen des Trägermediums führt. Um einer Überhitzung des Mediums entgegenzuwirken muss hier die Kühlung bei verlängerter bzw. intensiver Bestrahlung aktiv werden.

In einer weiteren Ausführungsform fungiert die elektromagnetische Strahlungsquelle nicht nur im Rahmen der NO-Generierung, sondern auch als Heizquelle einer Temperiervorrichtung.

Als zentraler Bestandteil der erfindungsgemäßen Vorrichtung ist die SGE ein offenes System oder dicht abgeschlossen. Die SGE ist dadurch gekennzeichnet, dass sie mit der gesamten Vorrichtung mechanisch fest verbunden ist oder lose an sie gebunden und leicht austauschbar sein kann, sodass sie erst vor dem Gebrauch der Vorrichtung in die Gesamtapparatur eingesetzt wird.

Ein weiteres Charakteristikum der SGE der erfindungsgemäßen Vorrichtung ist, dass in eine SGE auswechselbare bzw. austauschbare volumenfassende Füllbehälter (z. B. Austauschbehälter, Einsätze, Kartuschen, Pads, usw.; im folgenden als **FB** = *Füllbehälter* bezeichnet) eingesetzt werden können, die vorzugsweise chemisch stabile oder stabilisierte, potentiell NO-speichernde und somit potentiell wieder NO-freisetzende Substanzen (z. B. organische oder anorganische Nitrate, Nitrite, S-, N- oder O-Nitrosoverbindungen, NO-chelatierende Substanzen) allein oder in unterschiedlichen Kombinationen, welche in reiner Form oder gelöst in unterschiedlichen Lösungsmitteln, in einer katalysierten oder nicht katalysierten, physikalisch gestarteten und/oder chemischen und/oder enzymatischen Reaktion in der SGE NO freisetzen können, welches mit der genannten Pumpe oder auch ohne einer Umwälzvorrichtung direkt oder indirekt in das Volumen der Badelösung der VE eingeleitet werden kann.

In bevorzugter Weise handelt es sich bei dem austauschbaren Füllbehälter, der in die SGE und/oder in die VE eingesetzt werden kann, um eine Kartusche. Geeigneterweise enthält diese Kartusche eine pulverförmige, gelförmige oder flüssige Zusammensetzung umfassend NOD, Puffersubstanz, Antioxidans und optional ein Lösungsmittel.

Der Vorteil einer solchen Verwendung von auswechselbaren bzw. austauschbaren FBs ist, dass durch die Befüllung einer FB mit reaktiven Agenzien in unterschiedlichen Kombinationen und Konzentrationen die damit bestückte SGE bezüglich Länge und Konzentration unterschiedliche, charakteristische, benutzungs- und behandlungsspezifische NO-Freisetzungsmuster in den Badelösungen generieren könnte. Somit kann erreicht werden, dass die NO-Freisetzungs- bzw. Konzentrationsmuster in den Badelösungen durch die Wahl eines spezifisch befüllten und in die SGE eingesetzten FB eine an die Fach- und Verantwortungskompetenz des Endanwenders angepasste Anwendungsoptimierung ermöglicht. Was die Befüllung der FB betrifft, so werden dort Mengen von NO-Vorstufen (z. B. Nitrit oder S-Nitrosothiole) gewählt, die nach Auflösung der jeweiligen Substanz in der Badelösung zu Endkonzentrationen von vorzugsweise 0,001 bis 10000 mM, insbesondere 0,01 bis 6000 mM, besonders bevorzugt 0,1 bis 5000 mM, speziell 0,4 bis 2000 mM, ganz speziell 0,5 bis 1500 mM führen können.

Die NO-Erzeugung in der SGE der erfindungsgemäßen Vorrichtung wird vorzugsweise anhand der Manipulation verschiedener Einstellparameter geregelt werden. Dabei gelten als Einstellparameter die eingesetzten Konzentration der NO-freisetzenden Agenzien, die Stärke der elektromagnetischen Strahlung und die Eigenschaften der weiteren physikalischen und/oder chemischen Induktionsgrößen, welche für die NO-Freisetzung aus den Agenzien verantwortlich sind. Ferner können als mögliche Induktionsgrößen einer NO-Freisetzung aus potentiell NO-generierenden Substanzen einzeln oder in unterschiedlichen Kombinationen folgende Parameter variiert und genutzt werden:
- der pH-Wert,
- der Redoxstatus (Präsenz reduzierender oder oxidierender Stoffe),
- die Temperatur,
- Stromfluss und/oder Spannung,
- der umgebener Druck,
- die Intensität der elektromagnetischen Strahlung und Expositionsdauer, welcher die Badelösung in der SGE ausgesetzt wird,
- die exponierte Bestrahlungsfläche,
- die Zeit der Einwirkung einer Induktionsgröße auf die NO-freisetzenden Agenzien,
- die Strömungsgeschwindigkeit der Badelösung durch die SGE,
- die Entfernung der Quelle der elektromagnetischen Strahlung zur Reaktionslösung,
- das Spektrum der elektromagnetischen Strahlungsquelle,
- die Absorptions-, Transmissions-, Reflexionseigenschaften der Badelösung,
- oder die Konzentration von biologischen oder chemischen Katalysatoren oder Vermittlersubstanzen, die auch außerhalb der "typischen" physiko-chemischen Bedingungen einer optimalen NO-Freisetzung eine solche aus NO-generierenden Substanzen durch Katalyse oder entsprechende Akzeptoreigenschaften ermöglichen (z. B. mit Hilfe von Chromophoren und anderen Substanzen, mit deren Hilfe z. B. auch elektromagnetische Strahlung außerhalb des UVA-Spektrumbereiches in der Lage sein könnte, aus den entsprechenden NO-bildenden Agenzien eine NO-Freisetzung zu ermöglichen).

In Bezug auf den zuletzt genannten Punkt sei darauf hingewiesen, dass insbesondere in der Anwesenheit von Ionen von Übergangsmetallen, so z. B. Cu²⁺, wässrige Nitritlösungen bei wesentlich längeren Wellenlängen Licht absorbieren können als es reine Nitritlösungen tun und somit das Nitrition auch durch Licht in Wellenlängen von 400 - 450 nm und noch anderen Wellenlängen ≥ 450 nm unter NO-Freisetzung gespalten werden könnten. Zudem gilt, dass bedingt durch eine relativ schwache Bindungsenergie zwischen NO und dem Restmolekül S- und N-nitrosierte chemische Verbindungen ebenfalls durch elektromagnetische Strahlung ≥400 nm unter NO-Freisetzung photolytisch gespaltet werden können.

Die erfindungsgemäße Vorrichtung kann jedoch alternativ auch so konstruiert sein, dass auf die SGE mit den charakteristischen Funktionen gänzlich verzichtet wird und die für die NO-Generierung notwendige physikalisch/chemische Vorrichtung, z. B. die Lichtquelle mit den bereits genannten Eigenschaften ein fester Bestandteil der VE ist und somit die NO-haltigen Badelösungen direkt in der VE generiert werden und somit eine Umwälzung durch ein bestrahltes Volumen einer SGE gar nicht bedürfen. Die für den z. B. photolytischen Zerfall vorgesehene Strahlungsquelle kann daher außerhalb und/oder innerhalb einer VE so angebracht sein, dass die Strahlungsdurchflutung der VE mitsamt den das Stickstoffmonoxid freisetzenden Reaktionssubstanzen im Sinne eines induzierten Stoffzerfalls bzw. einer Freisetzung von Stickstoffmonoxid maximal bzw. optimal für die gewünschte Anwendung ist. Die Bestückung der VE mit photolabilen NO-Derivaten erfolgt entweder direkt, indem sie der Badelösung zugesetzt werden oder sie werden aus einer mit der VE assoziierten FB freigesetzt.

Auch hier emittiert die mit der VE assoziierte Strahlungsquelle vorzugsweise elektromagnetische Strahlung mit Wellenlängen von 100 bis 2000 nm oder elektromagnetische Strahlung jeder anderen Wellenlänget, die allein oder mit Unterstützung chemischer, physikalischer oder biologischer Verfahren eine Spaltung von Stickstoffmonoxid-Vorstufen induzieren und dadurch eine Freisetzung von Stickstoffmonoxid induzieren kann.

Die VE ist aus einem Material aufgebaut, das die Eigenschaften des für eine optimale Freisetzung von Stickstoffmonoxid notwendige Energie einer elektromagnetischen Strahlungsquelle nicht beeinflusst oder aufgrund seiner Eigenschaften die für eine lichtinduzierte Freisetzung von Stickstoffmonoxid notwendigen Lichteigenschaften erst schafft oder optimiert oder im Falle der pH-abhängigen NO-Generierung den pH-induzierten Nitritzerfall fördert und optimiert. Durch die Befüllung einer VE mit auswechselbaren bzw. austauschbaren Füllbehälter (FB), die mit Agenzien in unterschiedlichen Kombinationen und Konzentrationen befüllt sein können, kann die VE bezüglich Länge und Konzentration unterschiedliche, charakteristische und benutzungs- und behandlungsspezifische NO-Freisetzungsmuster in den Badelösungen generieren. Somit erlauben die NO-Freisetzungsmuster in den Badelösungen durch die Wahl eines spezifisch befüllten auswechselbaren bzw. austauschbaren und in die VE einsetzbaren FB eine Anwendungsoptimierung bzw. eine Anpassung an die Fach- und Verantwortungskompetenz des Endanwenders.

In der VE kann aus praktischen Gründen die Verwendung einer wässrigen Lösung einer Nitrit-Quelle bevorzugt sein, welche Konzentrationen von vorzugsweise 0,001 bis 10000 mM, insbesondere 0,01 bis 6000 mM, besonders bevorzugt 0,1 bis 5000 mM, speziell 0,4 bis 2000 mM, ganz speziell 0,5 bis 1500 mM, aufweisen.

In einer alternativen Ausführungsform weist die Nitrit-Quelle in der wässrigen Lösung der VE bevorzugt eine Konzentration von 100 µM bis 5000 mM, besonders bevorzugt von 500 µM bis 100 mM, und speziell von zwischen 1 mM bis 10 mM auf.

Daneben kann in der VE aus praktischen Gründen ebenfalls die Verwendung einer wässrigen Lösung mit einzelnen Stoffen oder auch Stoffgemischen aus der chemischen Familie der Nitrosoverbindungen (R-NO) bevorzugt werden, welche Konzentrationen von vorzugsweise 0,001 bis 10000 mM, insbesondere 0,01 bis 6000 mM, besonders bevorzugt 0,1 bis 5000 mM, speziell 0,4 bis 2000 mM, ganz speziell 0,5 bis 1500 mM, aufweisen.

In einer alternativen Ausführungsform weisen die Stoffen oder auch Stoffgemischen aus der chemischen Familie der Nitrosoverbindungen (R-NO) in der wässrigen Lösung der VE bevorzugt eine Konzentration von 100 µM bis 5000 mM, besonders bevorzugt von 500µM bis 2000 mM, und speziell von zwischen 10 mM bis 500 mM auf.

Zum Zwecke einer photolytisch induzierten NO-Bildung können in der VE, je nach gewünschter bzw. benötigter Höhe der in der Badelösung gelösten Konzentration des NO-Gases sowie je nach gewünschter bzw. benötigter Dauer des jeweiligen NO-Gehaltes der Badelösung, NO-generierende feste Stoffe in spezifischer Menge oder Lösungen mit Nitritsalzen oder den anderen oben erwähnten NO-generierenden Substanzen in den gewünschten bzw. angestrebten Konzentrationen verwendet werden.

Die NO-Erzeugung in der VE kann vorzugsweise anhand der Manipulation technischer, chemischer und physikalischer Einstellparameter geregelt werden. Als Einstellparameter kann die eingesetzten Konzentration der NO-freisetzenden Agenzien, die Stärke der elektromagnetischen Strahlung und die Eigenschaften der weiteren physikalischen und/oder chemischen Induktionsgrößen, welche für die NO-Freisetzung aus den Agenzien verantwortlich sind, genutzt werden. Dabei können als Induktionsgrößen einer NO-Freisetzung aus potentiell NO-generierenden Substanzen einzeln oder in unterschiedlichen Kombinationen Parameter variiert und genutzt werden, so z. B. der pH-Wert der Badelösung, der Redoxstatus der Badelösung (Präsenz reduzierender oder oxidierender Stoffe), die Temperatur der Badelösung, der umgebener Druck, die Intensität der elektromagnetischen Strahlung und Expositionsdauer, welcher die Badelösung in der VE ausgesetzt wird, die exponierte Bestrahlungsfläche, die Zeit der Einwirkung einer Induktionsgrößen auf die NO-freisetzenden Agenzien, die Strömungsgeschwindigkeit der Badelösung in der VE, die Entfernung der Quelle der elektromagnetischen Strahlung zur Reaktionslösung, das Spektrum der elektromagnetischen Strahlungsquelle, die Absorptions-, Transmissions-, Reflexionseigenschaften der Badelösung, die Konzentration von biologischen oder chemischen Katalysatoren oder Vermittlersubstanzen, die auch außerhalb der "typischen" physiko-chemischen Bedingungen einer optimalen NO-Freisetzung eine solche aus NO-generierenden Substanzen durch Katalyse oder entsprechende Akzeptoreigenschaften dennoch ermöglichen (z. B. mit Hilfe von Chromophoren und anderen Substanzen, mit deren Hilfe z. B. auch elektromagnetische Strahlung außerhalb des UV_{A}-Spektrumbereiches in der Lage sein könnte, aus den entsprechenden NO-bildenden Agenzien eine NO-Freisetzung zu ermöglichen).

Was die weiter oben angesprochenen Stellgrößen betrifft, so könnte bei der erfindungsgemäßen Vorrichtung bei einer konstant gehaltenen Induktionsgröße durch den Einsatz variierender Konzentrationen der Stickstoffoxid-freisetzenden Substanzen variierende Mengen an Stickstoffmonoxid erzeugt und in der Badelösung gelöst werden. Andererseits könnte bei einer konstanten Konzentration der Stickstoffoxid freisetzenden Substanz die Freisetzung von Stickstoffmonoxid in der Badelösung durch Variation der Einstellparameter der jeweiligen Induktionsgröße verändert werden.

Es ist bekannt, dass sich NO in wässrigen Lösungen bis zu einer Konzentration von ca. 2 mM lösen lässt, bevor es dann aus der Lösung in die umgebene Luft entweichen könnte. Um die Lösungskonzentration und/oder auch Lebensdauer von NO in einer wässrigen Badelösungen zu erhöhen, und um das Löslichkeitsverhalten von NO in der Badelösung zu steigern, kann die Badelösungen vor Gebrauch oder während des Gebrauchs mit inerten Gasen, z. B. Stickstoff (N₂), Helium (He) oder Argon (Ar) gesättigt werden. Zu diesem Zweck kann die erfindungsgemäße Vorrichtung zusätzlich über eine integrierte oder externe Apparatur verfügen, durch welche die Badelösung bei Bedarf geleitet wird und mit einem inerten Gas versetzt, angereichert und/oder gesättigt wird.

Entsprechend wird bei einer Ausführungsform der Erfindung das Trägermedium mit inerten Gasen angereichert werden, vorzugsweise mit Stickstoff, Helium oder Argon.

In einer bevorzugten Ausführungsform ist die NO-Konzentration des Trägermediums durch die maximale Löslichkeit des NO gegeben. Dies bedeutet, dass das NO gänzlich als gelöstes NO vorliegt und keine NO-Freisetzung, z.B. als NO-Gasbläschen auftritt. Neben der unnötigen Kontamination der Umwelt durch freisetzendes NO-Gas hat sich auch gezeigt, dass gasförmiges NO schlecht wieder im Trägermedium zu lösen ist.

In einem wässrigen Medium kann die maximale Löslichkeit von NO je nach Temperatur, pH-Wert und weiteren Bestandteilen zwischen 0,2 mM und 5 mM betragen.

An dieser Stelle sollte darauf hingewiesen werden, dass das als Träger für das NO benutzte Lösungsmedium der erfindungsgemäßen Vorrichtung nicht zwingend eine wässrige Lösung sein muss, sondern jedes andere organische oder anorganische, flüssige, zähe bis gelartige Trägermedium sein kann, welches in der Lage ist, NO zu speichern, aufzunehmen, zu lösen und eventuell wieder freigeben zu können.

Die mit Hilfe der hier beschriebenen erfindungsgemäßen Vorrichtung erzeugten NO-haltigen z. B. Badelösungen können aufgrund der physiologischen Rolle des NO zur Anregung des Stoffwechsels von Geweben durch äußerliche Anwendung, im Bereich der Dermatologie zur Behandlung von chirurgischen oder unfallbedingten Wunden, chronischen, nicht bzw. schlecht heilenden und/oder bakteriell befallenen Wunden sowie zur Behandlung von dermatologischen Erkrankungen aus dem Formenkreis der entzündlichen, immunologisch gesteuerten bzw. Autoimmunerkrankungen verwendet werden. Beispiele für mögliche Anwendungsgebiete wären:
- Anregung des Stoffwechsels von Geweben durch äußerliche Anwendung bei Mensch und Tier
- Behandlung diabetischer Füße und Wunden,
- Behandlung neuropathischer Schmerzen beim Diabetes und anderen Erkrankungen,
- Behandlung von Krampfadern,
- Behandlung lokaler oberflächlicher als auch tiefsitzender Ischämien und thrombopathischer Erkrankungen von Geweben,
- akute und chronische Entzündungen der Haut,
- Allergien der Haut,
- parasitäre Infektion der Haut,
- atopische Dermatitis insbesondere Neurodermitis,
- Dermatomyositis,
- Pemphigus vulgaris und/oder andere lokale und systemische Infektionen und/oder akute und chronische Entzündungssituationen,
- Wunddefekte, wie der chronische diabetisch-neuropathische Ulcus,
- Ulcus cruris,
- Dekubituswunden,
- sekundär heilende infizierte Wunden,
- reizlose, primär heilende Wunden, wie insbesondere ablative Riss- oder Schürfwunden,
- (Haut-)Transplantate,
- Behandlung des diabetischen Schmerzes der unteren Extremitäten (Fuß oder Bein); und
- Behandlung von schlecht perfundierten Lappenplastiken.

In einem weiteren Aspekt stellt die Erfindung ein Verfahren zur Behandlung eines Patienten bereit, das die folgenden Schritte umfasst:
a. Erzeugen eines mit NO angereicherten Trägermedium mittels der erfindungsgemäßen Eintauchvorrichtung; und
b. Eintauchen von Rumpfabschnitten, Extremitätenabschnitten oder des ganzen Körpers des Patienten in das Trägermedium der Eintauchvorrichtung.

Bevorzugterweise ist bei diesem Verfahren die Behandlung ausgewählt aus der Gruppe enthaltend:
µ Anregung des Stoffwechsels von Geweben durch äußerliche Anwendung bei Mensch und Tier;
• Behandlung von chirurgischen oder unfallbedingten Wunden;
• Behandlung von chronischen, nicht- oder schlecht-heilenden Wunden;
• Behandlung von bakteriell und/oder durch Pilze befallenen Wunden;
• Behandlung von dermatologischen Erkrankungen aus dem Formenkreis der entzündlichen, immunologisch gesteuerten oder Autoimmunerkrankungen;
• Behandlung diabetischer Füße und Wunden;
• Behandlung neuropathischer Schmerzen;
• Behandlung von Krampfadern;
• Behandlung von lokalen oberflächlichen oder tiefsitzenden Ischämien und thrombopathischer Erkrankungen von Geweben;
• Behandlung akuter und chronischer Entzündungen der Haut;
• Behandlung von Allergien der Haut;
• Behandlung von parasitären Infektionen der Haut;
• Behandlung der atopischen Dermatitis insbesondere Neurodermitis, Dermatomyositis und Pemphigus vulgaris;
• Behandlung von Wunddefekten, wie dem chronischen diabetischneuropathischen Ulcus, Ulcus cruris, Dekubituswunden;
• Behandlung größerer Körperareale zur Therapie systemischer Erkrankungen wie z. B. des erhöhten Blutdrucks (Hypertonie) und verwandten hämodynamischen Erkrankungen;
• Behandlung von Patienten mit (Haut)transplantaten;
• Behandlung des diabetischen Schmerzes der unteren Extremitäten (Fuß oder Bein); und
• Behandlung von schlecht perfundierten Lappenplastiken.

In einer bevorzugten Weise wird das Verfahren zur Behandlung chronischer Wunden der unteren Extremitäten von Diabetikern angewendet.

Zweckmäßigerweise ist das erfindungsgemäße Verfahren dadurch gekennzeichnet, dass die Behandlung durch Eintauchen des ganzen Körpers, Rumpfabschnitts oder Extremitätenabschnitts zwischen wenigen Sekunden und vielen Stunden dauern kann.

Bevorzugterweise erfolgt bei dem Verfahren die Behandlung durch Eintauchen des ganzen Körpers, Rumpfabschnitts oder Extremitätenabschnitts in dem Trägermedium für 5 bis 30 Minuten, bevorzugt für 7,5 bis 20 Minuten und besonders bevorzugt für 10 bis 15 Minuten.

In einer besonders bevorzugten Weise wird die erfindungsgemäße Eintauchvorrichtung zur Behandlung chronischer Wunden der unteren Extremitäten und hierbei insbesondere bei Diabetikern eingesetzt. Hierbei kann zudem durch die Behandlung im Sinne einer Prophylaxe das Entstehungsrisiko chronischer Wunden sowie die Anzahl medizinischer Amputationen verringert werden. Dadurch gehen die Reduktion der neuropathischen Beinschmerzen und die Herstellung eines verbesserten Wundmilieus mit einer spürbar verbesserten Lebensqualität der Patienten einher. Darüber hinaus ist durch eine Verkürzung der Wundversorgung eine signifikante Minderung der Behandlungskosten zu erwarten.

In einer Offenbarungsform der Erfindung wird die Eintauchvorrichtung zur Therapie schlechtheilender Wunden eingesetzt. Eine gestörte arterielle Durchblutung und/oder venöse Rückflussstörungen sind maßgebliche Ursachen in der Entstehung sowie Chronizität von Wunden der unteren Extremitäten. Eine NO-bedingte arterielle Vasodilatation verbessert die Durchblutung des betroffenen Gewebes und durch die antithrombogene Wirkung des NO wird ein venöser Rückfluss des Blutes wesentlich gefördert bzw. erleichtert. Die NO-abhängige Verbesserung beider hämodynamischer Parameter stellt den entscheidenden therapierelevanten Aspekt einer lokalen sowie systemischen Wirkung, die das Risiko der Entstehung von Wunden signifikant mindert bzw. deren Heilung wesentlich beschleunigt. Das mittels der Eintauchvorrichtung über das Trägermedium (Eintauchmedium) dem zu behandelnden ganzen Körper, dem Extremitätenabschnitt oder dem Rumpfabschnitt zugeführte NO kann daher erfolgreich zur Therapie schlechtheilender Wunden angewendet werden.

In einer besonderen Offenbarungsform wird die erfindungsgemäße Eintauchvorrichtung zur Behandlung des diabetischen Schmerz der unteren Extremitäten, also von Fuß und/oder Bein, eingesetzt. Der diabetische Schmerz ist ein sehr häufiges Ereignis im Verlauf einer Diabeteserkrankung. Der diabetischer Fuß-/Beinschmerz ist ein Ergebnis langandauernder erhöhter Blutglukosekonzentrationen, die die Grundursache für die während einer Diabeteserkrankung beobachteten Nerven- sowie Gefäßschädigung ist. Eine NO-bedingte arterielle Vasodilatation verbessert die Durchblutung des betroffenen Gewebes und hilft die Schmerzweiterleitung im Sinne einer Schmerzminderung zu beeinflussen. Das mit dem Trägermedium (Eintauchmedium) von außen dem Fuß und/oder Bein zugeführte NO kann daher erfolgreich zur Therapie des diabetischen Fuß-/Beinschmerz angewendet werden.

In einer speziellen Offenbarungsform der Erfindung wird die erfindungsgemäße Eintauchvorrichtung zur Behandlung von Patienten mit (Haut)transplantaten und hierbei insbesondere zur Behandlung von schlecht perfundierten Lappenplastiken eingesetzt. Die beiden vorab genannten hämodynamischen Größen, die arterielle Durchblutung sowie der venöse Rückfluss, stellen auch essenzielle Parameter des Therapieerfolgs chirurgischer Lappenplastiken dar. Als Lappenplastiken werden operative plastisch-chirurgische Techniken bezeichnet, die Haut und/oder Gewebe von einer (entbehrlichen) Stelle des gleichen Individuums an eine neue gewünschte Stelle bringen. In der Regel handelt es sich um reine Hautlappen, es kann aber jedes Gewebe mit oder ohne Haut sowohl gestielt (also mit seinen zugehörigen blutversorgenden Gefäßen und Nerven) als auch frei (d. h. mit Anschluss der Blutgefäße an die Blutversorgung der neuen Umgebung) verpflanzt werden. Die funktionelle Akzeptanz des verpflanzten Gewebes ist dabei ausschließlich von der arteriellen Blutversorgung sowie einem geregelten venösem Abfluss abhängig. Eine NO-bedingte arterielle Vasodilatation verbessert die Durchblutung und somit die notwendige Versorgung der Lappenplastik und durch die antithrombogene Wirkung des NO wird ein venöser Abfluss bzw. Rückfluss des Blutes gefördert und erleichtert. Von außen eingesetzte NO-Präparate können daher den Erfolg einer auf Lappenplastik basierten Therapieoption sichern bzw. fördern.

Zudem könnte es möglich sein, durch Behandlung größerer Körperareale auch systemische Erkrankungen, wie z. B. den erhöhten Blutdruck (Hypertonie) und verwandte hämodynamische Erkrankungen zu adressieren.

Daneben kann die erfindungsgemäße Vorrichtung auch zur Erzeugung NO-gesättigter Flüssigkeiten genutzt werden, die systemisch z. B. zur Behandlung von Bluthochdruck eingesetzt werden können, es kann zur Herstellung von nitrosierten Träger genutzt werden, die spontan wieder NO freisetzen und z. B. atopisch im Rahmen dermatologischer Therapien eingesetzt werden könnten, es könnte auch zur Herstellung unterschiedlicher NO-bindender Substanzen (z. B. NO-Donoren) genutzt werden.

Somit können mit der erfindungsgemäßen Vorrichtung auch Flüssigkeiten, Gele oder feste Stoffe als Träger-, Bindungs- oder Transportmedien für das NO dienen. Es ist denkbar, mit NO-Gas gesättigte Flüssigkeiten wie Puffer, Lösungen, Medien, Seren oder Blut als Trägermedium zu verwenden und diese z. B. im Rahmen einer Therapie lokal anzuwenden oder systemisch in die Blutbahn einzuleiten oder NO-gesättigte, zähflüssige Trägermedien wie Gele atopisch zu Behandlung von Wunden zu verwenden.

Zum Zweck der Behandlung werden zu behandelnde Areale oder Objekte in die Badelösung für die entsprechende Behandlungszeit, die zwischen wenigen Sekunden und vielen Stunden dauern kann, eingetaucht. Zum Zwecke einer besseren Vermischung der Badelösung kann die erfindungsgemäße Vorrichtung über eine Umwälzvorrichtung verfügen, die in die Gesamtvorrichtung integriert oder auch extern angebracht sein kann. Der Eintauchvorgang erfolgt dabei vorzugsweise in eine nach oben offene VE, wobei die obere Öffnung alternativ durch eine Dichtung oder Aufsatz abgedichtet werden kann, wobei jedoch das Einführen eines Objektes durch die Abdichtung möglich sein kann.

Um mögliche Verunreinigungen der Umgebungsluft mit NO oder dessen oxidativen Reaktionsprodukten zu mindern, zu vermeiden bzw. zu verhindern, kann die nach oben offene VE Variante über eine Vorrichtung verfügen, die über der Badelösung einen permanenten leichten Luftstrom generiert, der ebenfalls permanent absaugt und durch einen Aktivkohlefilter geleitet wird oder durch jede andere Vorrichtung, die in der Lage ist, entsprechende reaktive Gasspezies zu neutralisieren oder zu eliminieren. Diesem Gedanken folgend kann dementsprechend die nach oben abgedichtete Variante der VE über eine Vorrichtung verfügen, die das Gasvolumen oberhalb der Badelösung permanent absaugt, und ebenfalls durch einen Aktivkohlefilter oder entsprechend andere Vorrichtung nach außen leitet. So kann eine permanente Inaktivierung reaktiver Stickoxidspezies gewährleistet werden. Der dafür vorgesehene Aktivkohlefilter bzw. entsprechende anders technisch geartete Vorrichtung kann dabei eine auswechselbare und erneuerbare Einheit der VE sein.

Zum Zwecke einer sicheren Anwendung der erfindungsgemäßen Vorrichtung, wird diese über eine elektronisch gesteuerte, anwendungsspezifische Programmwahl inklusive einer Sicherheitsabschaltung des Gerätes, über entsprechende NO-, NO₂-, Temperatur- und Sicherheitssensorik sowie Fernbedienung und Verbindungsmöglichkeit an externe Steuerungs- und Dokumentationsgeräte bzw. -applikationen verfügen. Zum Sicherheitsmanagement zählt auch eine anwendungs- und benutzerspezifische und elektronisch gesteuerte Erkennung der spezifisch befüllten auswechselbaren bzw. austauschbaren Füllbehälter (FB), Umwälz- als auch Filtervorrichtung.

### BEISPIELE

### 1. Erzeugung von NO mittels der erfindungsgemäßen Vorrichtung

Die Stickstoffmonoxid-generierende Einheit enthielt Leitungswasser, Puffersalze (8 g/L NaCl + 0,2 g/L KCl + 1,424 g/L Na₂HPO₄ + 0,2 g/L KH₂PO₄, pH=7.0), 1,45 mM NaNO₂ und 10 mM Na-Askorbat.

Durch UV-Bestrahlung dieser Nitrit-haltigen Lösung wurde eine Lösung erzeugt, die ca. 150 µM NO enthielt. Anhand dieser Lösung wurden die Stabilität des erzeugten NOs und das Auftreten von Oxidationsprodukten analysiert. Weiterhin wurde die Wirkung bei einem Einsatz als Fußbad in Bezug auf Durchblutung und Erythembildung getestet.

### 1.1. Stabilitätstest

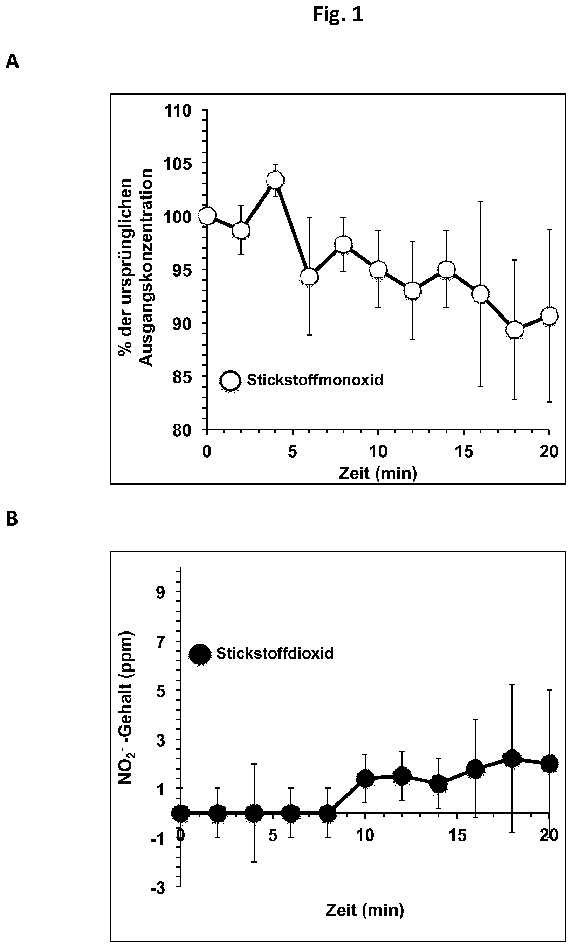

Dieser Lösung wurde zunächst einem Stabilitätstest unterzogen, d.h. es wurde ermittelt wie der Gehalt an NO sich in einem Zeitraum von 20 Minuten verändert. Alle 2 Minuten wurde ein Aliquot von 10 ml der Badelösung in einen mit Heliumgas (100 ml/min) durchströmenden Kolben (175 ml Fassungsvermögen) pipettiert, der bereits mit 40 ml PBS-Lösung (pH 7,4) befüllt war. Während der gesamten Prozedur wurden die aus dem Kolben ausweichenden Helium-Gas-Gemische direkt in eine NO-Analyseeinheit (Chemiluminescence Detector - CLD 822 Sr von Eco Physics, Duernten, Schweiz) geleitet, die die im Gasgemisch enthaltene NO-Menge in ppm (parts per million) detektierte und aufzeichnete (siehe dazu auch Opländer et al. 2010-Nitric Oxide-Bio. Ch. 23: 275-283). Die Messergebnisse sind in Figur 1A wiedergegeben. Es zeigte sich, dass der NO-Gehalt über den Zeitraum von 20 Minuten nahezu konstant war, er nahm statistisch nicht signifikant um etwa 10% ab.

### 1.2. Generierung von Oxidationsprodukten

Anhand der oben beschrieben Lösung wurde parallel zu der oben erwähnten NO-Detektion während derselben Messung die Menge von Stickstoffdioxidradikalen als wichtigstes Oxidationsprodukt des NO detektiert. Hier traten erst nach 10 Minuten sehr geringe Konzentrationen an NO₂ im Bereich von 1.5 ppm auf, die bis zu dem maximalen Zeitwert von 20 Minuten nur unwesentlich anstiegen. Die gemessenen NO₂-Werte waren jedoch nicht signifikant höher als die Ausgangswerte (siehe Figur 1B).

### 1.3 Verwendung als Fußbad

In die oben beschriebene Lösung (27°C), die eine konstante Menge NO enthielt, wurde ein Fuß des freiwilligen Probanden eingetaucht und in diesem Fußbad für insgesamt 10 Minuten belassen. Während des Fußbades wurde die Lösung durch leichte Bewegung des eingetauchten Fußes vorsichtig agitiert. Der Fuß wurde während dieses 10 minütigen Expositionsintervals und den anschließenden 15 Minuten hinsichtlich Hautdurchblutung in einer Gewebetiefe von 1-2 mm und 6-8 mm analysiert. Zudem wurde der Fuß auf Erythembildung hin untersucht.

### 1.3.1 Dermale Durchblutung

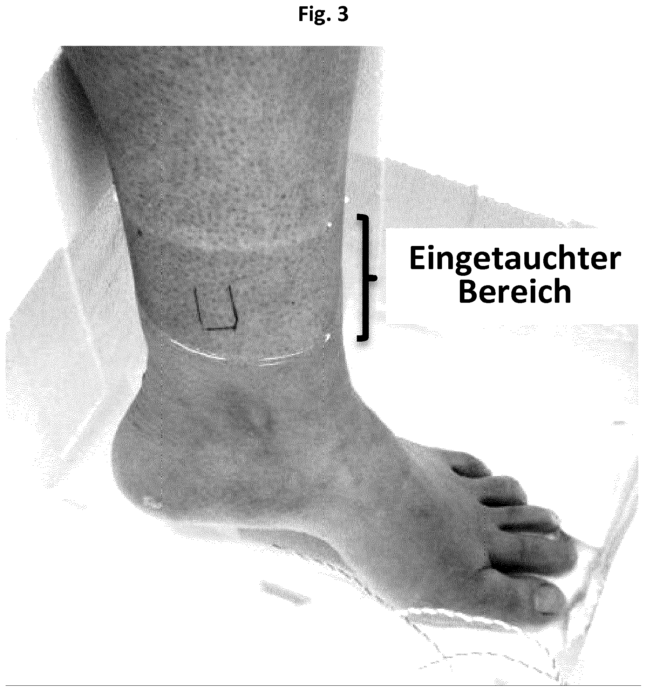

Bei beiden Hauttiefen wurde ein signifikanter Anstieg der Durchblutung durch die 10-minütige Exposition in dem NO-Fußbad beobachtet (siehe Figur 2A und 2B), um dann in einem Zeitraum von 10 bis 15 Minuten wieder auf Kontrollwerte zurückzukehren. Die erhöhte Hautdurchblutung konnte auch mit bloßem Auge durch Ausbildung eines temporären Erythems nachvollzogen werden (s. Figur 3).

### FIGUREN

Die Erfindung wird nachfolgend anhand der Figuren näher erläutert ohne die Erfindung auf dieses zu beschränken. Es zeigen:
- Fig. 1A:: das Ergebnis einer Stabilitätsmessung einer NO-haltigen gepufferten wässrigen Lösung gemäß Beispiel 1 über eine Zeitspanne von 20 Minuten. Dargestellt ist der Anteil in % bezogen auf den ursprünglichen NO-Gehalt.
- Fig. 1B:: das Ergebnis einer Analyse des Oxidationsprodukts NO₂⁻ in einer NO-haltigen gepufferten wässrigen Lösung gemäß Beispiel 1über eine Zeitspanne von 20 Minuten. Dargestellt ist der Gehalt an NO₂⁻ in parts per million (ppm).
- Fig. 2:: die Bestimmung der Hautdurchblutung während einer 10 minütigen Exposition durch Eintauchen eines Fußes in ein NO-haltiges Fußbad und einer anschließenden Zeitspanne von 15 Minuten (s. Beispiel 1). Dargestellt ist das relative Verhältnis der Durchblutung in eine beliebigen Einheit (arbitrary unit; AU) sowohl bei einer Gewebetiefe von 1 bis 2 mm (A) als auch einer Gewebetiefe von 6 bis 8 mm (B). Hierbei wurde der Fuß entweder in einer gepufferten NO-haltigen Lösung (weiße Quadrate) oder als Kontrolle in einer entsprechenden Pufferlösung (schwarzen Raute) gebadet und die Durchblutung nicht-invasiv unter Verwendung einer Flachsonde und des O2C-Doppler-spektroskopischen Detektionssystems (LEA-Medizintechnik GmbH, Gießen, BRD) zu den angegeben Zeitpunkten erfasst.
- Fig. 3:: den Nachweis der erhöhten Hautdurchblutung nach 10 minütiger Exposition eines Fußes in einem NO-haltiges Fußbad durch (temporäre) Erythembildung. Auf dem Foto ist deutlich sichtbar, dass der Fuß in dem eingetauchten Bereich aufgrund der Mehrdurchblutung stark gerötet ist.

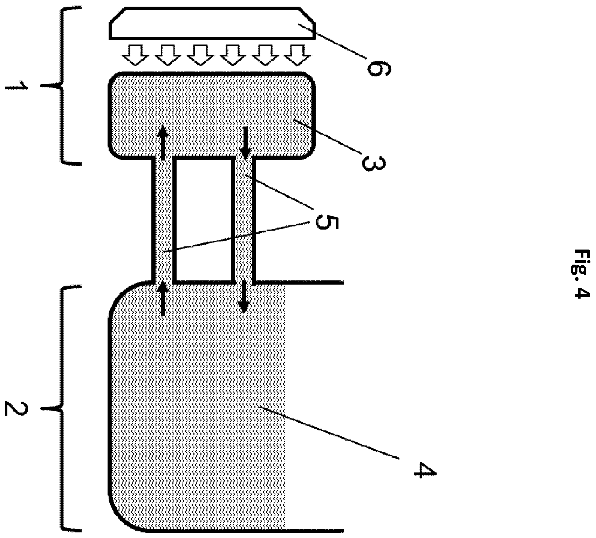

- Fig. 4:: die schematische Darstellung einer Eintauchvorrichtung im Querschnitt mit der Stickstoffmonoxid-generierenden Einheit (SGE) (1) und der Volumeneinheit (VE) (2), die durch zwei Verbindungsrohre miteinander verbunden sind (5). Die nach oben geöffnete VE enthält ein Eintauchmedium (4) zum Eintauchen von Objekten. Die SGE umfasst eine Volumenkammer mit Trägermedium (3), die von einer UV-Strahlungsquelle (6) bestrahlt wird.

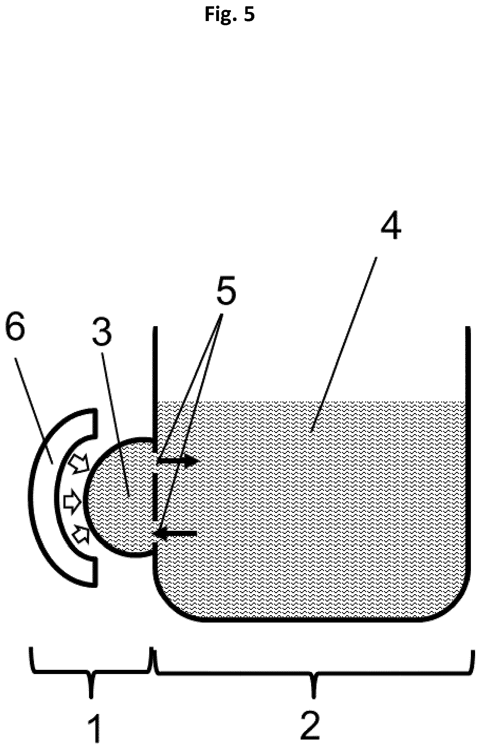

- Fig. 5:: die schematische Darstellung einer Eintauchvorrichtung im Querschnitt mit der Stickstoffmonoxid-generierenden Einheit (SGE) (1) und der Volumeneinheit (VE) (2), wobei die SGE direkt an der VE befestigt ist und durch zwei Öffnungen eine Verbindung zwischen diesen Kompartimenten besteht (5). Die nach oben geöffnete VE enthält ein Eintauchmedium (4) zum Eintauchen von Objekten. Die SGE umfasst eine Volumenkammer mit Trägermedium (3), die von einer UV-Strahlungsquelle (6) bestrahlt wird.

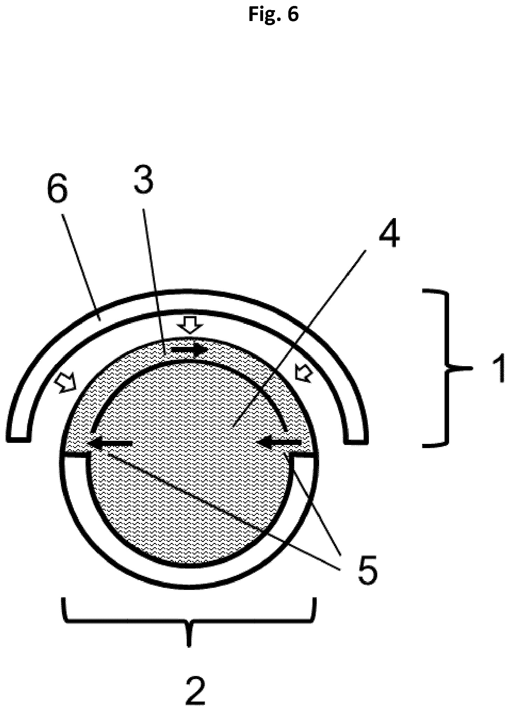

- Fig. 6:: die schematische Darstellung einer Eintauchvorrichtung im Querschnitt von oben gesehen mit der Stickstoffmonoxid-generierenden Einheit (SGE) (1) und der Volumeneinheit (VE) (2), wobei die SGE und die VE innerhalb eines kreisrunden Gefäßes angeordnet sind und die SGE als Außensegment von der VE abgetrennt ist. Die SGE steht hierbei durch zwei Öffnungen (5) mit der VE in Verbindung. Die VE als innerer Bereich des kreisrunden Gefäßes ist mit dem Eintauchmedium (4) zum Eintauchen von Objekten gefüllt. Das in der Volumenkammer der SGE enthaltene Trägermedium (3) wird von einer gekrümmten außenliegenden UV-Strahlungsquelle (6) bestrahlt.

### LITERATUR

[1] K.D. Kröncke, K. Fehsel, and V. Kolb-Bachofen, Inducible nitric oxide synthase in human diseases. Clin Exp Immunol 113 (1998) 147-56.
[2] K. Matsunaga, and R.F. Furchgott, Responses of rabbit aorta to nitric oxide and superoxide generated by ultraviolet irradiation of solutions containing inorganic nitrite. J Pharmacol Exp Ther 259 (1991) 1140-6.
[3] M. Fischer, and P. Warneck, Photodecomposition of nitrite and undissociated nitrous acid in aqueous solution. J.Phys.Chem. 100 (1996) 18749-18756.
[4] H. Strehlow, and I. Wagner, Flash photolysis in aqueous nitrite solutions. Z. Phys. Chem. NF 132 (1982) 151-160.
[5] S. Frank, H. Kampfer, C. Wetzler, and J. Pfeilschifter, Nitric oxide drives skin repair: novel functions of an established mediator. Kidney Int 61 (2002) 882-8.
[6] S. Frank, B. Stallmeyer, H. Kampfer, N. Kolb, and J. Pfeilschifter, Nitric oxide triggers enhanced induction of vascular endothelial growth factor expression in cultured keratinocytes (HaCaT) and during cutaneous wound repair. Faseb J 13 (1999) 2002-14.
[7] S. Frank, H. Kampfer, M. Podda, R. Kaufmann, and J. Pfeilschifter, Identification of copper/zinc superoxide dismutase as a nitric oxide- regulated gene in human (HaCaT) keratinocytes: implications for keratinocyte proliferation. Biochem J 346 Pt 3 (2000) 719-28.
[8] K. Yamasaki, H.D. Edington, C. McClosky, E. Tzeng, A. Lizonova, I. Kovesdi, D.L. Steed, and T.R. Billiar, Reversal of impaired wound repair in iNOS-deficient mice by topical adenoviral-mediated iNOS gene transfer. J Clin Invest 101 (1998) 967-71.
[9] J. Pfeilschifter, W. Eberhardt, and A. Huwiler, Nitric oxide and mechanisms of redox signalling: matrix and matrix-metabolizing enzymes as prime nitric oxide targets. Eur J Pharmacol 429 (2001) 279-86.
[10] Y. Ishii, T. Ogura, M. Tatemichi, H. Fujisawa, F. Otsuka, and H. Esumi, Induction of matrix metalloproteinase gene transcription by nitric oxide and mechanisms of MMP-1 gene induction in human melanoma cell lines. Int J Cancer 103 (2003) 161-8.
[11] M.B. Witte, F.J. Thornton, D.T. Efron, and A. Barbul, Enhancement of fibroblast collagen synthesis by nitric oxide. Nitric Oxide 4 (2000) 572-82.
[12] F. Verrecchia, and A. Mauviel, TGF-beta and TNF-alpha: antagonistic cytokines controlling type I collagen gene expression. Cell Signal 16 (2004) 873-80.
[13] D.A. Siwik, and W.S. Colucci, Regulation of matrix metalloproteinases by cytokines and reactive oxygen/nitrogen species in the myocardium. Heart Fail Rev 9 (2004) 43-51.
[14] V.M. Darley-Usmar, R.P. Patel, V.B. O'Donnell, and B.A. Freeman, Antioxidant actions of nitric oxide. in: L.J. Ignarro, (Ed.), Nitric Oxide: Biology and Pathobiology, Academic Press, San Diego, 2000, pp. 265-276.
[15] S.P. Goss, B. Kalyanaraman, and N. Hogg, Antioxidant effects of nitric oxide and nitric oxide donor compounds on low-density lipoprotein oxidation. Methods Enzymol 301 (1999) 444-53.
[16] D.A. Wink, J.A. Cook, R. Pacelli, J. Liebmann, M.C. Krishna, and J.B. Mitchell, Nitric oxide (NO) protects against cellular damage by reactive oxygen species. Toxicol Lett 82-83 (1995) 221-6.
[17] B. Brüne, A. von Knethen, and K.B. Sandau, Nitric oxide (NO): an effector of apoptosis. Cell Death Differ 6 (1999) 969-75.
[18] D. Moellering, J. McAndrew, R.P. Patel, T. Cornwell, T. Lincoln, X. Cao, J.L. Messina, H.J. Forman, H. Jo, and V.M. Darley-Usmar, Nitric oxide-dependent induction of glutathione synthesis through increased expression of gamma-glutamylcysteine synthetase. Arch Biochem Biophys 358 (1998) 74-82.
[19] U. Forstermann, M. Nakane, W.R. Tracey, and J.S. Pollock, Isoforms of nitric oxide synthase: functions in the cardiovascular system. Eur Heart J 14 Suppl I (1993) 10-5.
[20] P. He, M. Zeng, and F.E. Curry, Effect of nitric oxide synthase inhibitors on basal microvessel permeability and endothelial cell [Ca2+]i. Am J Physiol 273 (1997) H747-55.
[21] M. Toborek, and S. Kaiser, Endothelial cell functions. Relationship to atherogenesis. Basic Res Cardiol 94 (1999) 295-314.
[22] M. Keim, and B. Strauer, Endotheliale Dysfunktion; Therapeutische und prognostische Relevanz. Internist. 40 (1999) 1300-1307.
[23] T.P. Amadeu, A.B. Seabra, M.G. de Oliveira, and A.M. Costa, S-nitrosoglutathionecontaining hydrogel accelerates rat cutaneous wound repair. J Eur Acad Dermatol Venereol 21 (2007) 629-37.
[24] R. Weller, and M.J. Finnen, The effects of topical treatment with acidified nitrite on wound healing in normal and diabetic mice. Nitric Oxide 15 (2006) 395-9.
[25] A.B. Shekhter, V.A. Serezhenkov, T.G. Rudenko, A.V. Pekshev, and A.F. Vanin, Beneficial effect of gaseous nitric oxide on the healing of skin wounds. Nitric Oxide 12 (2005) 210-9.
[26] W.S. McDonald, T.P. Lo, Jr., M. Thurmond, C. Jones, R. Cohen, A. Miller, and D. Beasley, Role of nitric oxide in skin flap delay. Plast Reconstr Surg 113 (2004) 927-31.
[27] C. Beige, P.B. Massion, M. Pelat, and J.L. Balligand, Nitric oxide and the heart: update on new paradigms. Ann N Y Acad Sci 1047 (2005) 173-82.
[28] B. Gaston, Summary: systemic effects of inhaled nitric oxide. Proc Am Thorac Soc 3 (2006) 170-2.
[29] T.M. Dawson, and S.H. Snyder, Gases as biological messengers: nitric oxide and carbon monoxide in the brain. J Neurosci 14 (1994) 5147-59.
[30] C.C. Miller, M.K. Miller, A. Ghaffari, and B. Kunimoto, Treatment of chronic nonhealing leg ulceration with gaseous nitric oxide: a case study. J Cutan Med Surg 8 (2004) 233-8.
[31] A. Ghaffari, D.H. Neil, A. Ardakani, J. Road, A. Ghahary, and C.C. Miller, A direct nitric oxide gas delivery system for bacterial and mammalian cell cultures. Nitric Oxide 12 (2005) 129-40.
[32] A. Ghaffari, C.C. Miller, B. McMullin, and A. Ghahary, Potential application of gaseous nitric oxide as a topical antimicrobial agent. Nitric Oxide 14 (2006) 21-9.
[33] A. Ghaffari, R. Jalili, M. Ghaffari, C. Miller, and A. Ghahary, Efficacy of gaseous nitric oxide in the treatment of skin and soft tissue infections. Wound Repair Regen 15 (2007) 368-77.
[34] Z.S. Galis, and J.J. Khatri, Matrix metalloproteinases in vascular remodeling and atherogenesis: the good, the bad, and the ugly. Circ Res 90 (2002) 251-62.
[35] S.C. Tyagi, and M.R. Hayden, Role of nitric oxide in matrix remodeling in diabetes and heart failure. Heart Fail Rev 8 (2003) 23-8.
[36] J. Pfeilschifter, W. Eberhardt, and K.F. Beck, Regulation of gene expression by nitric oxide. Pflugers Arch 442 (2001) 479-86.
[37] R. Zamora, Y. Vodovotz, K.S. Aulak, P.K. Kim, J.M. Kane, 3rd, L. Alarcon, D.J. Stuehr, and T.R. Billiar, A DNA microarray study of nitric oxide-induced genes in mouse hepatocytes: implications for hepatic heme oxygenase-1 expression in ischemia/reperfusion. Nitric Oxide 7 (2002) 165-86.
[38] J. Hemish, N. Nakaya, V. Mittal, and G. Enikolopov, Nitric oxide activates diverse signaling pathways to regulate gene expression. J Biol Chem 278 (2003) 42321-9.
[39] M. Ziche, L. Morbidelli, E. Masini, S. Amerini, H.J. Granger, C.A. Maggi, P. Geppetti, and F. Ledda, Nitric oxide mediates angiogenesis in vivo and endothelial cell growth and migration in vitro promoted by substance P. J Clin Invest 94 (1994) 2036-44.
[40] S.J. Leibovich, P.J. Polverini, T.W. Fong, L.A. Harlow, and A.E. Koch, Production of angiogenic activity by human monocytes requires an L-arginine/nitric oxide-synthasedependent effector mechanism. Proc Natl Acad Sci U S A 91 (1994) 4190-4.
[41] N.S. Bryan, B.O. Fernandez, S.M. Bauer, M.F. Garcia-Saura, A.B. Milsom, T. Rassaf, R.E. Maloney, A. Bharti, J. Rodriguez, and M. Feelisch, Nitrite is a signaling molecule and regulator of gene expression in mammalian tissues. Nature Chemical Biology 1 (2005) 290-297.

### Zusammenfassung

Die vorliegende Erfindung betrifft eine Eintauchvorrichtung, die eine Stickstoffmonoxid-generierende Einheit und einer zum Eintauchen von Objekten vorgesehene Volumeneinheit umfasst, die Verwendung dieser Vorrichtung zur Behandlung von Erkrankungen, insbesondere von chronischen Wunden und Diabetes- sowie Gefäßerkrankungs-assoziierten Durchblutungsstörungen.

## Patentansprüche

1. Eintauchvorrichtung bestehend aus
a. einer Stickstoffmonoxid (NO) - generierenden Einheit (SGE) (1) mit einer UV-Strahlungsquelle (6) und einer Volumenkammer (3) zur Aufnahme einer NODenthaltenden wässrigen Lösung, wobei die SGE so ausgestaltet ist, dass mittels UV-Strahlung die wässrige Lösung mit NO angereichert werden kann, und
b. einer Volumeneinheit VE (2) zum Eintauchen von Objekten, wobei die VE die mit NO angereicherte, von der SGE (1) transferierte wässrige Lösung aufnehmen kann,
wobei die Volumeneinheit (2) ein nach oben offener Behälter ist, und die SGE (1) dicht verschließbar ist, **dadurch gekennzeichnet, dass**
der Transfer der wässrigen Lösung durch zwei, die SGE und VE verbindende Öffnungen (5) in einer gemeinsamen Wand oder durch zwei Leitungen erfolgt und die wässrige Lösung mit Hilfe einer Umwälz- bzw. Pumpvorrichtung durch die Volumenkammer (3) der SGE (1) geleitet werden kann und wieder in die VE (2) ausgeleitet werden kann, so dass die Volumenkammer (3) der SGE (1) und die Volumeneinheit (2) einen geschlossenen Kreislauf für die wässrige Lösung bilden, und
wobei die Eintauchvorrichtung mit einer Temperiervorrichtung versehen ist, die durch Heizen oder Abkühlen eine Einstellung einer ausgewählten Temperatur erlaubt, und
wobei die UV-Strahlungsquelle (6) ausgewählt ist aus der Gruppe enthaltend eine mit entsprechenden Fluorochromen beschichtete Glüh- oder Gasentladungslampe (niedrigdruck- oder hochdruckentladend), eine Licht-emittierende Diode (LED), eine organische Licht-emittierende Diode (OLED), und ein Laser.

2. Eintauchvorrichtung gemäß Anspruch 1, **dadurch gekennzeichnet, dass** in die Volumenkammer (3) der SGE (1) und/oder in die Volumeneinheit VE (2) ein austauschbarer Füllbehälter eingesetzt werden kann, der eine pulverförmige, gelförmige oder flüssige Zusammensetzung umfassend NOD, Puffersubstanz, Antioxidans und optional ein Lösungsmittel enthält.

3. Eintauchvorrichtung gemäß Anspruch 1 oder 2, **dadurch gekennzeichnet, dass** der austauschbare Füllbehälter eine Kartusche ist.

4. Eintauchvorrichtung gemäß einem der Ansprüche 1 bis 3 zur Verwendung bei der Behandlung von Erkrankungen mittels Eintauchen von Rumpfabschnitten, Extremitätenabschnitten oder ganzen Körpern als Objekten in das Trägermedium.

5. Eintauchvorrichtung gemäß Anspruch 4, **dadurch gekennzeichnet, dass** die SGE (1) so angesteuert wird, dass der Gehalt an NO über den Zeitraum der Behandlung ansteigt oder abfällt.

## Claims

1. Immersion device consisting of
a) unit (SGE) generating nitric oxide (NO) with a UV radiation source (6) and a volume chamber (3) for the reception of an NOD-containing aqueous solution, wherein the SGE is configured in such a manner that by means of UV radiation the aqueous solution can be enriched with NO, and
b) a volume unit VE (2) for the immersion of objects, wherein the VE can receive the NO- enriched aqueous solution transferred by the SGE (1),
wherein the volume unit (2) is a container, which is open towards the top, and the SGE (1) can be tightly sealed, **characterised in that**
the transfer of the aqueous solution occurs via two openings (5) connecting the SGE and VE in a common wall, or via two conduits and the aqueous solution can be guided with the aid of a circulating or pump device through the volume chamber (3) of the SGE (1) and can be diverted into the VE (2), so that the volume chamber (3) of the SGE (1) and the volume unit (2) form a closed circuit for the aqueous solution, and
wherein the immersion device is provided with a temperature control device, which allows a setting of a selected temperature through heating or cooling, and
wherein the UV radiation source (6) is selected from a group containing a glow or gas discharge lamp (low-pressure or high-pressure discharge) coated with corresponding fluorochromes, a light-emitting diode (LED), an organic light-emitting diode (OLED), and a laser.

2. Immersion device according to claim 1, **characterised in that** in the volume chamber (3) of the SGE (1) and/or in the volume unit VE (2) an exchangeable filling container can be inserted, which contains a powder-like, gel-like or liquid composition including NOD, buffer substance, antioxidant, and optionally a solvent.

3. Immersion device according to claim 1 or 2, **characterised in that** the exchangeable filling container is a cartridge.

4. Immersion device according to one of the claims 1 to 3 for use in the treatment of illnesses by means of immersion of body sections, extremity sections or whole bodies as objects into the carrier medium.

5. Immersion device according to claim 4, **characterised in that** the SGE (1) is controlled in such a way that the amount of NO increases or decreases during the period of the treatment.

## Revendications

1. Dispositif d'immersion constitué
a. d'une unité (SGE) (1) générant de l'oxyde nitrique (NO), comprenant une source de rayonnement UV (6) et une chambre volumique (3) destinée à réceptionner une solution aqueuse contenant NOD, l'unité SGE étant conçue de manière à ce que la solution aqueuse puisse être enrichie au moyen du rayonnement UV, et
b. d'une unité volumique VE (2) destinée à immerger des objets, l'unité VE pouvant réceptionner la solution aqueuse enrichie en NO, transférée par l'unité SGE (1),
l'unité volumique (2) étant un récipient ouvert vers le haut, et l'unité SGE (1) pouvant être fermée de manière étanche, **caractérisé en ce que**
le transfert de la solution aqueuse étant réalisé à travers deux ouvertures (5) reliant l'unité SGE à l'unité VE dans une paroi commune ou au moyen de deux conduites, et la solution aqueuse pouvant être guidée à travers la chambre volumique (3) de l'unité SGE (1) à l'aide d'un dispositif de circulation ou de pompage et pouvant être de nouveau amenée dans l'unité VE (2) de sorte que la chambre volumique (3) de l'unité SGE (1) et l'unité volumique (2) forment un circuit fermé pour la solution aqueuse, et
le dispositif d'immersion étant muni d'un dispositif de régulation de température, lequel, par échauffement ou refroidissement, permet un réglage d'une température sélectionnée, et
la source de rayonnement UV (6) étant sélectionnée dans le groupe contenant une lampe à incandescence ou lampe à décharge de gaz (déchargeant à basse ou à haute pression) revêtue de fluorochromes correspondants, une diode (LED) émettant de la lumière, une diode organique (OLED) émettant de la lumière, et un laser.

2. Dispositif d'immersion selon la revendication 1, **caractérisé en ce qu'**un récipient de remplissage interchangeable peut être inséré dans la chambre volumique (3) de l'unité SGE (1) et/ou dans la chambre volumique VE (2), lequel contient une composition sous forme de poudre, sous forme de gel ou liquide, comprenant NOD, une substance tampon, des antioxydants et un solvant optionnellement.

3. Dispositif d'immersion selon la revendication 1 ou 2, **caractérisé en ce que** le récipient de remplissage interchangeable est une cartouche.

4. Dispositif d'immersion selon l'une quelconque des revendications 1 à 3, destiné à être utilisé lors du traitement de maladies par immersion de sections du torse, de sections des extrémités ou de corps entiers en tant qu'objets dans le milieu porteur.

5. Dispositif d'immersion selon la revendication 4, **caractérisé en ce que** l'unité SGE (1) est commandée de manière à ce que la teneur en NO augmente ou baisse pendant la période du traitement.
